# EUROPEAN PATENT APPLICATION

(11) **EP 4 661 024 A2**
(43) Date of publication of application: **10.12.2025**
(21) Application number: 25190969.3
(22) Date of filing: 01.06.2022
(51) Int. Cl.: G16H 50/00

(54) **AUTOMATED MANAGEMENT OF CLINICAL MODIFICATIONS TO TREATMENT PLANS USING THREE-DIMENSIONAL CONTROLS**

(30) Priority: 01.06.2021 US 202163195674 P
(62) Divisional of application: 22733820.9
(71) Applicant: Align Technology, Inc., San Jose, CA 95134 (US)
(72) Inventor: BLANCO, Samuel, San Jose, 95134 (US); FLANAGAN, Michael, San Jose, 95134 (US); MEYER, Eric P., San Jose, 95134 (US); LOGVANEV, Igor, San Jose, 95134 (US); MEDNIKOV, Dmitry, San Jose, 95134 (US); PORYVKIN, Alexander, San Jose, 95134 (US); MAKAROV, Sergey, San Jose, 95134 (US); IVANOV, Vasily, San Jose, 95134 (US)
(74) Representative: Carpmaels & Ransford LLP

(57) **Abstract**

Methods and apparatuses, including systems, for forming and modifying treatment plans, including modifying treatment plan in real time. In particular are methods and apparatuses for modifying and orthodontic treatment plan in real time that may include the use of an enhanced user interface including a 3D user overlay.

## Description

### CLAIM OF PRIORITY

This patent application claims priority to U.S. Provisional Patent Application No. 63/195,674, titled "AUTOMATED MANAGEMENT OF CLINICAL MODIFICATIONS TO TREATMENT PLANS USING THREE-DIMENSIONAL CONTROLS," filed on June 1, 2021, and herein incorporated by reference in its entirety.

### INCORPORATION BY REFERENCE

All publications and patent applications mentioned in this specification are herein incorporated by reference in their entirety to the same extent as if each individual publication or patent application was specifically and individually indicated to be incorporated by reference.

### BACKGROUND

Orthodontic and dental treatments using a series of patient-removable appliances (e.g., "aligners") are very useful for treating patients, and in particular for treating malocclusions. Treatment planning is typically performed in conjunction with the dental professional (e.g., dentist, orthodontist, dental technician, etc.), by generating a model of the patient's teeth in a final configuration and then breaking the treatment plan into a number of intermediate stages (steps) corresponding to individual appliances that are worn sequentially. This process may be interactive, adjusting the staging and in some cases the final target position, based on constraints on the movement of the teeth and the dental professional's preferences. Once the final treatment plan is finalized, the series of aligners may be manufactured corresponding to the treatment planning.

This treatment planning process may include many manual steps that are complex and may require a high level of knowledge of orthodontic norms. Further, because the steps are performed in series, the process may require a substantial amount of time. Manual steps may include preparation of the model for digital planning, reviewing and modifying proposed treatment plans (including staging) and aligner features placement (which includes features placed either on a tooth or on an aligner itself). These steps may be performed before providing an initial treatment plan to a dental professional, who may then modify the plan further and send it back for additional processing to adjust the treatment plan, repeating (iterating) this process until a final treatment plan is completed and then provided to the patient.

However, the additional manual processing when modifying the plan may add delay to the overall workflow, up to several weeks in some instances. For instance, a dental professional may send instructions to a dental technician for manually modifying the treatment plan. These instructions may be added to the technician's queue such that the technician may not quickly turnaround the modified plan. In addition, when the dental professional receives the modified plan for approval, the dental professional may require additional time to recall the patient and the intended treatment, further reducing efficiency.

What is needed are apparatuses (e.g., system and devices, including software) and methods that may improve treatment planning, including potentially increasing the speed at which treatment plans may be completed, as well as providing greater choices and control to the dental professional. The methods and apparatuses described herein may address these needs.

### SUMMARY OF THE DISCLOSURE

Described herein are apparatuses (e.g., systems and devices, including software) and methods for automated management of clinical modifications to treatment plans using three-dimensional controls.

The systems and methods described herein may improve the functioning of a computing device by reducing computing resources and overhead for transmitting and/or storing updated treatment planning data, thereby improving processing efficiency of the computing device over conventional approaches. These systems and methods may also improve the field of orthodontic treatment by improving the efficiency of treatment planning and reducing a time to reach a fabrication stage. Moreover, the systems and methods provided herein may improve the field of medical care by improving a digital workflow procedure by reducing costs of human time spent on processing, increasing efficiency via automation, and reducing potential errors.

A "dental consumer," as used herein, may include a person seeking assessment, diagnosis, and/or treatment for a dental condition (general dental condition, orthodontic condition, endodontic condition, condition requiring restorative dentistry, etc.). A dental consumer may, but need not, have agreed to and/or started treatment for a dental condition. A "dental patient" (used interchangeably with patient herein) as used herein, may include a person who has agreed to diagnosis and/or treatment for a dental condition. A dental consumer and/or a dental patient, may, for instance, be interested in and/or have started orthodontic treatment, such as treatment using one or more (e.g., a sequence of) aligners (e.g., polymeric appliances having a plurality of tooth-receiving cavities shaped to successively reposition a person's teeth from an initial arrangement toward a target arrangement).

A "dental professional" (used interchangeably with dentist, orthodontist, and doctor herein) as used herein, may include any person with specialized training in the field of dentistry, and may include, without limitation, general practice dentists, orthodontists, dental technicians, dental hygienists, etc. A dental professional may include a person who can assess, diagnose, and/or treat a dental condition. "Assessment" of a dental condition, as used herein, may include an estimation of the existence of a dental condition. An assessment of a dental condition need not be a clinical diagnosis of the dental condition. In some embodiments, an "assessment" of a dental condition may include an "image based assessment," that is an assessment of a dental condition based in part or on whole on photos and/or images (e.g., images that are not used to stitch a mesh or form the basis of a clinical scan) taken of the dental condition. A "diagnosis" of a dental condition, as used herein, may include a clinical identification of the nature of an illness or other problem by examination of the symptoms. "Treatment" of a dental condition, as used herein, may include prescription and/or administration of care to address the dental conditions. Examples of treatments to dental conditions include prescription and/or administration of brackets/wires, clear aligners, and/or other appliances to address orthodontic conditions, prescription and/or administration of restorative elements to address bring dentition to functional and/or aesthetic requirements, etc.

For example, described herein are methods comprising: generating a digital model of a final position of a patient's teeth from a scan of the patient's teeth in an initial position of the patient's teeth; generating a treatment plan comprising incremental positions of the patient's teeth to move the patient's teeth from the initial position towards the final position; providing a three-dimensional representation of the treatment plan to a display; receiving, in real time, a user request to modify the treatment plan and determining, in real time, that the requested user modification is within a predetermined thresholds for modifications to the treatment plan, generating, automatically and in real time when the user requested modification is within the predetermined threshold, a revised treatment plan based on the user requested modification; and outputting to the display a three-dimensional representation of the revised treatment plan.

These methods may be methods of orthodontically treating a patient's teeth.

Any of the methods described herein may include determining, in real time, that the requested user modification is within the predetermined thresholds for modifications to the treatment plan comprises determining that the user requested modification is within a predetermined threshold for space required for one or more of: attachments and power ridges, or moving teeth without collisions.

Generating the treatment plan may comprise generating the treatment plan at a remote processor and transferring the treatment plan to a local processor that is in immediate communication with the display. The local processor may be referred to herein as a "backend" processor or backend system. By "immediate communication" the local processor may directly control the output to the display; in some examples the local processor is local to the display or in direct communication with the display. Generating the revised treatment plan may comprise generating the treatment plan at the local processor. Any of these methods may include transferring the revised treatment plan to the remote processor for quality review.

Any of the methods and apparatuses described herein may include scanning a patient's teeth to generating a model of an initial position of the patient's teeth. For example, the patient's teeth may be scanned by an intraoral scanner and the scanner may be in communication with the local processor directly or indirectly.

Any of these methods may include overlaying three-dimensional controls over the treatment plan on the display. In some examples the methods or apparatuses may include receiving the user request to modify the treatment plan via the three-dimensional controls, wherein the request to modify the treatment plan is based on the modification received via the three-dimensional controls. In some examples the user request to modify the treatment plan is received via text instructions or an IPL compatible input.

Any of these methods may include returning an indication to the display if the user request to modify the treatment plan is outside of the predetermined thresholds for modifications to the treatment plan. The predetermined thresholds for modifications to the treatment plan may include tooth movement thresholds. In some examples the predetermined thresholds for modifications to the treatment plan are location thresholds for aligner features. In some examples the location threshold is a proximity threshold with respect to a gingival line. The indication may include a visual indication of the location threshold on the display. The aligner features may include one or more of: an attachment, a cut, a hook, or power ridges. The modification may be one or more of: a modification to a tooth location, interproximal reduction amount, tooth shape, pontic shape, gingiva shape, the final position of the patient's teeth, intermediate positions of the patient's teeth in one or more stages of the treatment plan, to locations of or stages used for attachments, locations of or stages used for power ridges, locations of or stages used for hooks, locations of or stages used for precision cuts, or locations of or stages used for interproximal reduction.

Any of these methods may include outputting instructions for fabricating a plurality of orthodontic appliances based on the modified treatment plan. Any of these methods may include forming one or more aligners from the modified treatment plan.

In any of these methods generating, automatically and in real time may include generating within 15 minutes or less (e.g., 12 minutes or less, 10 minutes or less, 8 minutes or less, 7 minutes or less, 6 minutes or less, 5 minutes or less, 4 minutes or less, 3 minutes or less, 2 minutes or less, 1 minute or less, etc.) of receiving the user request to modify the treatment plan.

For example, a method of orthodontically treating teeth may include: generating a digital model of a final position of a patient's teeth from a scan of the patient's teeth in an initial position of the patient's teeth; generating, at a remote processor, a treatment plan comprising incremental positions of the patient's teeth to move the patient's teeth from the initial position towards the final position, and transferring the treatment plan to a local processor; providing a three-dimensional representation of the treatment plan to a display that is in immediate communication with the local processor; receiving, in real time at the remote processor, a user request to modify the treatment plan and determining, in real time at the remote processor, that the requested user modification is within a predetermined thresholds for modifications to the treatment plan, generating, at the remote processor and in real time when the user requested modification is within the predetermined threshold, a revised treatment plan based on the user requested modification; and outputting to the display a three-dimensional representation of the revised treatment plan.

Any of the methods (and method steps) described herein may be implemented by a system. For example, A system (e.g., for orthodontically treating teeth) may include: one or more processors and memory comprising instructions that when executed by the one more processors causes the system perform any of these methods, including: generate a digital model of a final position of a patient's teeth from a scan of the patient's teeth in an initial position of the patient's teeth; generate a treatment plan comprising incremental positions of the patient's teeth to move the patient's teeth from the initial position towards the final position; provide a three-dimensional representation of the treatment plan to a display; receive, in real time, a user request to modify the treatment plan and determine, in real time, that the requested user modification is within a predetermined thresholds for modifications to the treatment plan, generate, automatically and in real time when the user requested modification is within the predetermined threshold, a revised treatment plan based on the user requested modification; and output to the display a three-dimensional representation of the revised treatment plan.

In any of these apparatuses and methods the treatment plan may comprise incremental positions of the patient's teeth to move the patient's teeth from the initial position towards the final position. Generating the revised treatment may include generating one or more of a revised tooth shape, restorative object shape, implant shape, location, or orientation, or auxiliary component generation.

Determining that the requested user modification is within the predetermined thresholds for modifications to the treatment plan may comprise determining that the user requested modification is within a predetermined threshold for space required for one or more of: attachments and power ridges, or moving teeth without collisions.

Generating the treatment plan may comprise generating the treatment plan at a remote processor and transferring the treatment plan to a local processor that is in immediate communication with the display. For example, generating the revised treatment plan may comprise generating the treatment plan at the local processor.

Any of these methods may include transferring the revised treatment plan to the remote processor for quality review. The instructions may further cause the system to scan a patient's teeth to generating a model of an initial position of the patient's teeth. In some examples, the instructions may further cause the system to overlay three-dimensional controls over the treatment plan on the display. In some examples, the instructions further cause the system to receive the user request to modify the treatment plan via the three-dimensional controls, wherein the user request to modify the treatment plan is based on the modification received via the three-dimensional controls. The user request to modify the treatment plan may be received via text instructions or an IPL compatible input. The instructions may further cause the system to return an indication to the display if the user request to modify the treatment plan is outside of the predetermined thresholds for modifications to the treatment plan. The predetermined thresholds for modifications to the treatment plan may be tooth movement thresholds. In some examples the predetermined thresholds for modifications to the treatment plan are location thresholds for aligner features. The location threshold may be a proximity threshold with respect to a gingival line. The indication may include a visual indication of the location threshold on the display. The aligner features may be one or more of: an attachment, a cut, a hook, or power ridges. The modification may be one or more of: a modification to a tooth location, interproximal reduction amount, tooth shape, pontic shape, gingiva shape, the final position of the patient's teeth, intermediate positions of the patient's teeth in one or more stages of the treatment plan, to locations of or stages used for attachments, locations of or stages used for power ridges, locations of or stages used for hooks, locations of or stages used for precision cuts, or locations of or stages used for interproximal reduction.

The instructions may further cause the system to output instructions for fabricating a plurality of orthodontic appliances based on the modified treatment plan.

For example, a system for orthodontically treating teeth may include: one or more processors and memory comprising instructions that when executed by the one more processors causes the system to: generate a digital model of a final position of a patient's teeth from a scan of the patient's teeth in an initial position of the patient's teeth; generate, at a remote processor, a treatment plan comprising incremental positions of the patient's teeth to move the patient's teeth from the initial position towards the final position, and transfer the treatment plan to a local processor; provide a three-dimensional representation of the treatment plan to a display that is in immediate communication with the local processor; receive, in real time at the remote processor, a user request to modify the treatment plan and determine, in real time at the remote processor, that the requested user modification is within a predetermined thresholds for modifications to the treatment plan, generate, at the remote processor and in real time when the user requested modification is within the predetermined threshold, a revised treatment plan based on the user requested modification; and output to the display a three-dimensional representation of the revised treatment plan.

All of the methods and apparatuses described herein, in any combination, are herein contemplated and can be used to achieve the benefits as described herein.

### BRIEF DESCRIPTION OF THE DRAWINGS

A better understanding of the features and advantages of the methods and apparatuses described herein will be obtained by reference to the following detailed description that sets forth illustrative embodiments, and the accompanying drawings of which:
FIG. 1A illustrates an exemplary tooth repositioning appliance or aligner that can be worn by a patient in order to achieve an incremental repositioning of individual teeth in the jaw, in accordance with some embodiments.
FIG. 1B illustrates a tooth repositioning system, in accordance with some embodiments.
FIG. 2 shows a system for simulating and planning an orthodontic treatment, in accordance with some embodiments.
FIG. 3 illustrates an exemplary workflow for automated management of clinical modifications to treatment plans using three-dimensional controls, in accordance with some embodiments.
FIGS. 4A-B illustrate another exemplary workflow for automated management of clinical modifications to treatment plans using three-dimensional controls, in accordance with some embodiments.
FIG. 5 illustrates an example interface loading a treatment plan and tools for a new modification, in accordance with some embodiments.
FIG. 6 illustrates an example interface selecting a tooth for modification, in accordance with some embodiments.
FIG. 7 illustrates an example interface selecting a feature for modification, in accordance with some embodiments.
FIG. 8 illustrates an example interface dragging a selected feature, in accordance with some embodiments.
FIG. 9 illustrates an example interface displaying constraints, in accordance with some embodiments.
FIG. 10 illustrates an example side-by-side comparison of an initial treatment plan and updated treatment plan, in accordance with some embodiments.
FIG. 11 illustrates an example dataflow for automated management of clinical modifications to treatment plans, in accordance with some embodiments.
FIG. 12 shows a method of automated management of clinical modifications to treatment plans using three-dimensional controls, in accordance with some embodiments.
FIG. 13 shows a block diagram of an example computing system capable of implementing one or more embodiments described and/or illustrated herein, in accordance with some embodiments.
FIG. 14 shows a block diagram of an example computing network capable of implementing one or more of the embodiments described and/or illustrated herein, in accordance with some embodiments.

### DETAILED DESCRIPTION

The following detailed description and provides a better understanding of the features and advantages of the inventions described in the present disclosure in accordance with the embodiments disclosed herein. Although the detailed description includes many specific embodiments, these are provided by way of example only and should not be construed as limiting the scope of the inventions disclosed herein.

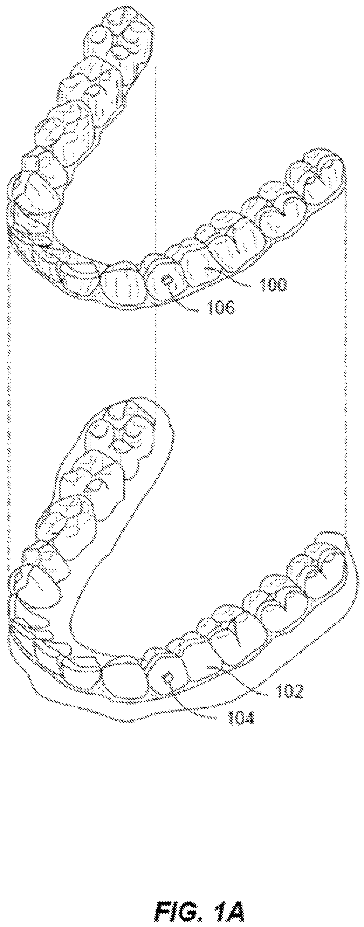

FIG. 1A illustrates an exemplary tooth repositioning appliance 100, such as an aligner that can be worn by a patient in order to achieve an incremental repositioning of individual teeth 102 in the jaw. The appliance can include a shell (e.g., a continuous polymeric shell or a segmented shell) having teeth-receiving cavities that receive and resiliently reposition the teeth. An appliance or portion(s) thereof may be indirectly fabricated using a physical model of teeth. For example, an appliance (e.g., polymeric appliance) can be formed using a physical model of teeth and a sheet of suitable layers of polymeric material. The physical model (e.g., physical mold) of teeth can be formed through a variety of techniques, including 3D printing. The appliance can be formed by thermoforming the appliance over the physical model. In some embodiments, a physical appliance is directly fabricated, e.g., using additive manufacturing techniques, from a digital model of an appliance. In some embodiments, the physical appliance may be created through a variety of direct formation techniques, such as 3D printing. An appliance can fit over all teeth present in an upper or lower jaw, or less than all of the teeth. The appliance can be designed specifically to accommodate the teeth of the patient (e.g., the topography of the tooth-receiving cavities matches the topography of the patient's teeth), and may be fabricated based on positive or negative models of the patient's teeth generated by impression, scanning, and the like. Alternatively, the appliance can be a generic appliance configured to receive the teeth, but not necessarily shaped to match the topography of the patient's teeth. In some cases, only certain teeth received by an appliance will be repositioned by the appliance while other teeth can provide a base or anchor region for holding the appliance in place as it applies force against the tooth or teeth targeted for repositioning. In some cases, some or most, and even all, of the teeth will be repositioned at some point during treatment. Teeth that are moved can also serve as a base or anchor for holding the appliance as it is worn by the patient. In some embodiments, no wires or other means will be provided for holding an appliance in place over the teeth. In some cases, however, it may be desirable or necessary to provide individual attachments or other anchoring elements 104 on teeth 102 with corresponding receptacles or apertures 106 in the appliance 100 so that the appliance can apply a selected force on the tooth. Exemplary appliances, including those utilized in the Invisalign^{®} System, are described in numerous patents and patent applications assigned to Align Technology, Inc. including, for example, in U.S. Pat. Nos. 6,450,807, and 5,975,893, as well as on the company's website, which is accessible on the World Wide Web (see, e.g., the URL "invisalign.com"). Examples of tooth-mounted attachments suitable for use with orthodontic appliances are also described in patents and patent applications assigned to Align Technology, Inc., including, for example, U.S. Pat. Nos. 6,309,215 and 6,830,450.

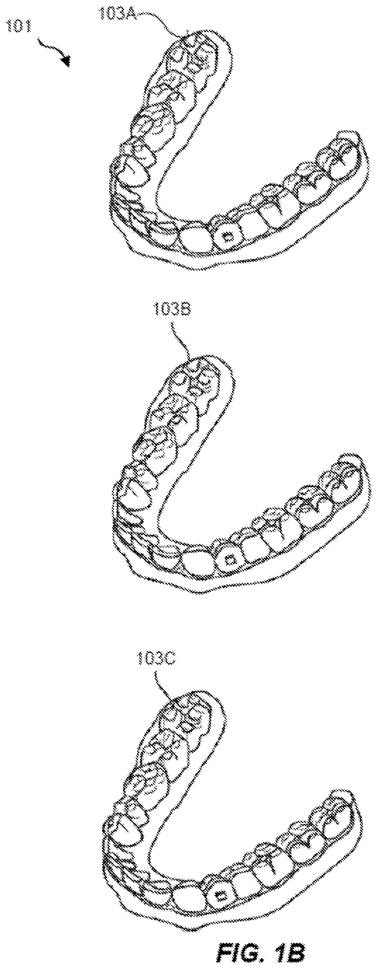

FIG. 1B illustrates a tooth repositioning system 101 including a plurality of appliances 103A, 103B, 103C. Any of the appliances described herein can be designed and/or provided as part of a set of a plurality of appliances used in a tooth repositioning system. Each appliance may be configured so a tooth-receiving cavity has a geometry corresponding to an intermediate or final tooth arrangement intended for the appliance. The patient's teeth can be progressively repositioned from an initial tooth arrangement to a target tooth arrangement by placing a series of incremental position adjustment appliances over the patient's teeth. For example, the tooth repositioning system 101 can include a first appliance 103A corresponding to an initial tooth arrangement, one or more intermediate appliances 103B corresponding to one or more intermediate arrangements, and a final appliance 103C corresponding to a target arrangement. A target tooth arrangement can be a planned final tooth arrangement selected for the patient's teeth at the end of all planned orthodontic treatment. Alternatively, a target arrangement can be one of some intermediate arrangements for the patient's teeth during the course of orthodontic treatment, which may include various different treatment scenarios, including, but not limited to, instances where surgery is recommended, where interproximal reduction (IPR) is appropriate, where a progress check is scheduled, where anchor placement is best, where palatal expansion is desirable, where restorative dentistry is involved (e.g., inlays, onlays, crowns, bridges, implants, veneers, and the like), etc. As such, it is understood that a target tooth arrangement can be any planned resulting arrangement for the patient's teeth that follows one or more incremental repositioning stages. Likewise, an initial tooth arrangement can be any initial arrangement for the patient's teeth that is followed by one or more incremental repositioning stages.

Optionally, in cases involving more complex movements or treatment plans, it may be beneficial to utilize auxiliary components (e.g., features, accessories, structures, devices, components, and the like) in conjunction with an orthodontic appliance. Examples of such accessories include but are not limited to elastics, wires, springs, bars, arch expanders, palatal expanders, twin blocks, occlusal blocks, bite ramps, mandibular advancement splints, bite plates, pontics, hooks, brackets, headgear tubes, springs, bumper tubes, palatal bars, frameworks, pin- and-tube apparatuses, buccal shields, buccinator bows, wire shields, lingual flanges and pads, lip pads or bumpers, protrusions, divots, and the like. In some embodiments, the appliances, systems and methods described herein include improved orthodontic appliances with integrally formed features that are shaped to couple to such auxiliary components, or that replace such auxiliary components.

In some cases, after an initial treatment plan is established, the doctor may determine that modifications to the treatment plan are necessary. In a typical workflow, the doctor may provide instructions to a technician for modifying the treatment plan, such as moving attachments, changing positions of teeth, and/or other changes to final positions, staging, aligner features, etc. The technician may receive these instructions but may not be immediately available to perform the modifications. For example, these instructions may be entered into a queue of different cases that the technician may be working through.

Once the technician applies the instructed modifications to the treatment plan and checks the updated treatment plan for constraint violations, warnings for the doctor to consider, errors in the information sent to the technician, or other errors, messages, or information, the technician may send the updated treatment plan back to the doctor for approval. As the doctor may receive the updated treatment plan days or weeks after, the doctor may require time to recall the patient and planned treatment, verify the updated treatment plan includes the needed modifications, and finally approve the updated treatment plan. This back and forth between the doctor and the technician may repeat for several iterations as needed until the doctor may finally approved the treatment plan.

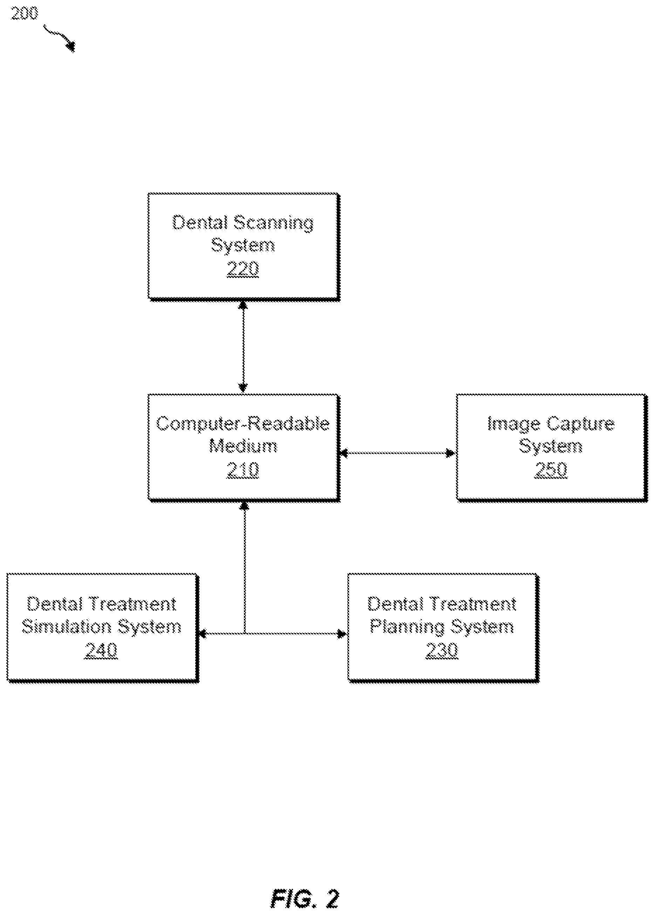

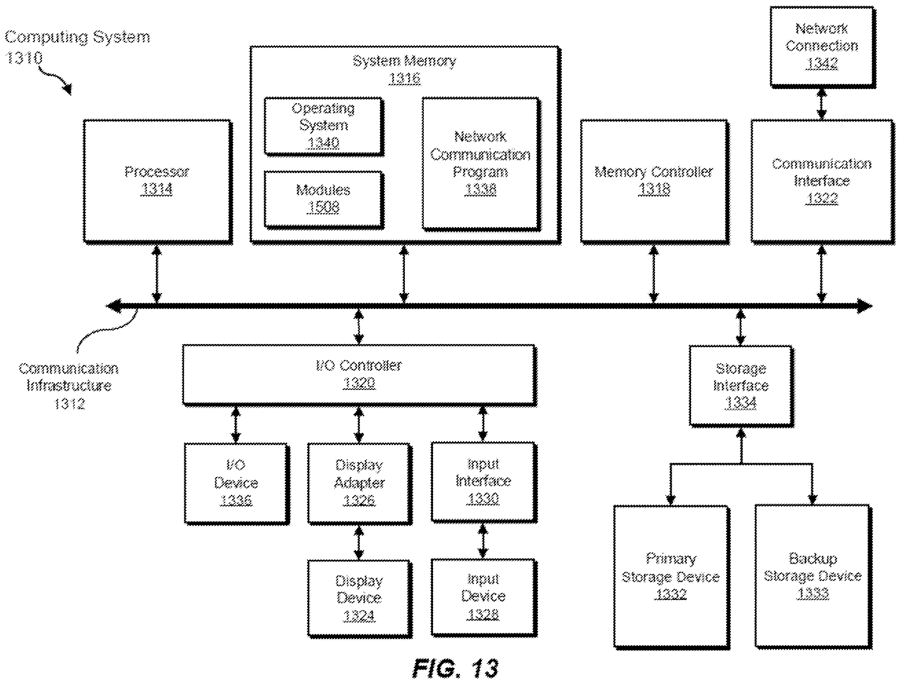

FIG. 2 shows an example of a system 200 for simulating and planning an orthodontic treatment, in accordance with some embodiments. In the example of FIG. 2, the system 200 includes a computer-readable medium 210, a dental scanning system 220, a dental treatment planning system 230, a dental treatment simulation system 240, and an image capture system 250. One or more of the elements of the system 200 may include elements of such as those described with reference to the computer system shown in FIG. 13, and vice versa. One or more elements of system 200 may also include one or more computer readable media including instructions that when executed by a processor, for example, a processor of any of systems 220, 230, 240, and 250 cause the respective system or systems to perform the processes described herein.

Dental scanning system 220 may include a computer system configured to capture one or more scans of a patient's dentition. Dental scanning system 220 may include a scan engine for capturing 2D or 3D images of a patient. Such images may include images of the patient's teeth, face, and jaw, for example. The images may also include x-rays, computed tomography, magnetic resonance imaging (MRI), cone beam computed tomography (CBCT), cephalogram images, panoramic x-ray images, digital imaging and communication in medicine (DICOM) images, or other subsurface images of the patient. The scan engine may also capture 3D data representing the patient's teeth, face, gingiva, or other aspects of the patient.

Dental scanning system 220 may also include a 2D imaging system, such as a still or video camera, an x-ray machine, or other 2D imager. In some embodiments, dental scanning system 220 may also include a 3D imager, such as an intraoral scanner, an impression scanner, a tomography system, a cone beam computed tomography (CBCT) system, or other system as described herein, for example. Dental scanning system 220 and associated engines and imagers can be used to capture the historic scan data for use in determining the historic mean parameters of a 3D parametric dental model, as described herein. Dental scanning system 220 and associated engines and imagers can be used to capture the 2D and 3D images of a patient's face and dentition for use in building a 3D parametric model of the patient's teeth as described herein. Examples of parametric models of the patient's teeth suitable for incorporation in accordance with the present disclosure are describe in U.S. Application No. 16/400,980, filed on May 1, 2019, entitled "Providing a simulated outcome of dental treatment on a patient", published as US20200000551on January 2, 2020, the entire disclosure of which is incorporated herein by reference.

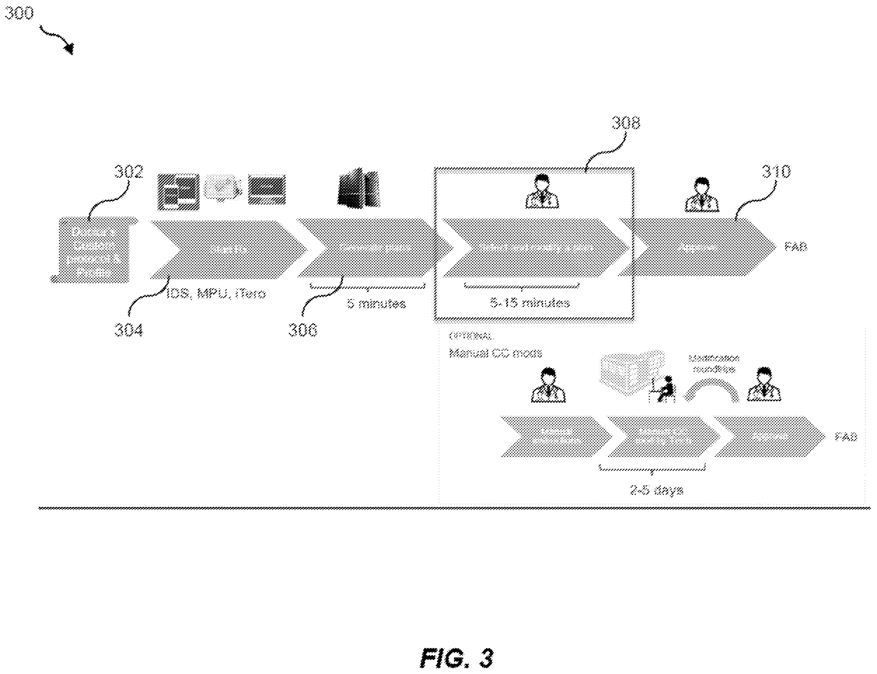

Dental treatment simulation system 240 may include a computer system configured to simulate one or more estimated and/or intended outcomes of a dental treatment plan. In some implementations, dental treatment simulation system 240 obtains photos and/or other 2D images of a consumer/patient. Dental treatment simulation system 240 may further be configured to determine tooth, lip, gingiva, and/or other edges related to teeth in the 2D image. As noted herein, dental treatment simulation system 240 may be configured to match tooth and/or arch parameters to tooth, lip, gingiva, and/or other edges. Dental treatment simulation system 240 may also render a 3D tooth model of the patient's teeth. Dental treatment simulation system 240 may gather information related to historical and/or idealized arches representing an estimated outcome of treatment. Dental treatment simulation system 240 may, in various implementations, insert, align, etc. the 3D tooth model with the 2D image of the patient in order to render a 2D simulation of an estimated outcome of orthodontic treatment. Dental treatment simulation system 240 may include a photo parameterization engine which may further include an edge analysis engine, an expectation-maximization (EM) analysis engine, a course tooth alignment engine, and a 3D parameterization conversion engine. The dental treatment simulation system 240 may also include a parametric treatment prediction engine which may further include a treatment parameterization engine, a scanned tooth normalization engine, and a treatment plan remodeling engine. Dental treatment simulation system 240 and its associated engines may carry out the processes described herein, for example with reference to FIGS. 3, 11, 13, and 14.

Dental treatment planning system 230 may include a computer system configured to implement treatment plans. Dental treatment planning system 230 may include a rendering engine and interface for visualizing or otherwise displaying the simulated outcome of the dental treatment plan. For example, the rendering engine may render the visualizations of the 3D models described herein. Dental treatment planning system 230 may also determine an orthodontic treatment plan for moving a patient's teeth from an initial position, for example, based in part on the 2D image of the patient's teeth, to a final position. Dental treatment planning system 230 may be operative to provide for image viewing and manipulation such that rendered images may be scrollable, pivotable, zoomable, and interactive. Dental treatment planning system 230 may include graphics rendering hardware, one or more displays, and one or more input devices. Some or all of dental treatment planning system 230 may be implemented on a personal computing device such as a desktop computing device or a handheld device, such as a mobile phone. In some embodiments, at least a portion of dental treatment planning system 230 may be implemented on a scanning system, such as dental scanning system 220. Image capture system 250 may include a device configured to obtain an image, including an image of a patient. The image capture system may comprise any type of mobile device (iOS devices, iPhones, iPads, iPods, etc., Android devices, portable devices, tablets), PCs, cameras (DSLR cameras, film cameras, video cameras, still cameras, etc.). In some implementations, image capture system 250 comprises a set of stored images, such as images stored on a storage device, a network location, a social media website, etc.

The systems and methods herein provide for a more efficient digital workflow for treatment planning. For example, in one scenario, a doctor may receive and view, via a frontend application and/or system, an initial treatment plan for a patient. The doctor may decide that the plan needs adjustments to final positions and/or changes to attachments. The doctor may then fine tune the final positions and/or change the attachments using 3D controls with the frontend application. The doctor may run a recalculation (e.g., a "live update") to incorporate the changes. This recalculation may have a faster turnaround than manual modification (e.g., in a matter of minutes rather than days or weeks). The doctor may view the updated treatment plan and approve.

In another scenario, the doctor may have gone through several iterations of modifications with a CAD designer and before final approval, may notice several attachments that should have been removed during the last round of modifications. The doctor may remove the attachments using the 3D controls with the frontend application, run the recalculation ("live update") and approve the modified treatment plan.

FIG. 3 illustrates a workflow 300 using any of the systems and/or methods described herein. At 302, a doctor may establish a custom protocol and profile. For example, the doctor may adjust a default protocol and/or profile for treatment planning, such as by modifying parameters relating to treatment. The custom protocol may include a staging pattern determined by the doctor. The staging pattern may define how many total stages for a treatment plan, and how many desired teeth to be moved in each stage. For example, a V pattern may start with moving a tooth in a primary stage, and moving immediately neighboring teeth on subsequent stages akin to a "V" shape branching from the first tooth.

At 304, a new treatment for a patient may be initiated, which may incorporate the doctor's custom protocol and/or profile. For example, the patient's teeth may be scanned, and the doctor may establish desired final positions of the patient's teeth.

At 306, an initial treatment plan may be generated based on the doctor's inputs at 302 and/or 304. The initial treatment plan may be generated by computer using patient scans and the doctor's inputs and may take, for example, 5 minutes to generate.

At 308, the doctor may select and modify a plan. The systems and methods described herein may provide an interface, which may include 3D controls, for modifying treatment plans. The modifications may include updates to the final attributes of the treatment plan, such as staging and/or appliance features (e.g., attachments, precision cuts, etc.). The doctor may apply, via the interface, the modifications such that the doctor may review the modified treatment plan near real time, without significant delay. Applying the modifications may include comparing the modifications with acceptable treatment thresholds for a treatment plan, translating the doctor's modifications to treatment operations prescribed by the treatment plan, and modifying the treatment plan based on the doctor's modifications. Modifying the treatment plan may, for example, take around 5-15 minutes. The doctor may repeat 308 to make additional modifications as needed.

At 310, the doctor may review the modified treatment plan and approve. Once approved, appliances may be fabricated according to the approved treatment plan.

Optionally at 308, the doctor may provide manual instructions for a technician to manually modify the treatment plan, similar to a conventional workflow. Several round trips of modifications between the doctor and technician may be needed before the doctor approves. However, as described herein, manual modifications may require significantly more time, such as 2-5 days.

As seen in workflow 300, by removing manual modification steps, the entire workflow may be completed in minutes rather than days or weeks. Advantageously, the reduced time may allow the doctor to finalize the patient's treatment plan in a single session rather than waiting days for the modified treatment plan having to recall the patient's case, further improving the doctor's efficiency. In addition, the doctor may be able to talk through a finalized treatment plan with the patient during an office visit. The doctor may be able to make "live updates" to the treatment plan until the patient is also satisfied with the treatment plan.

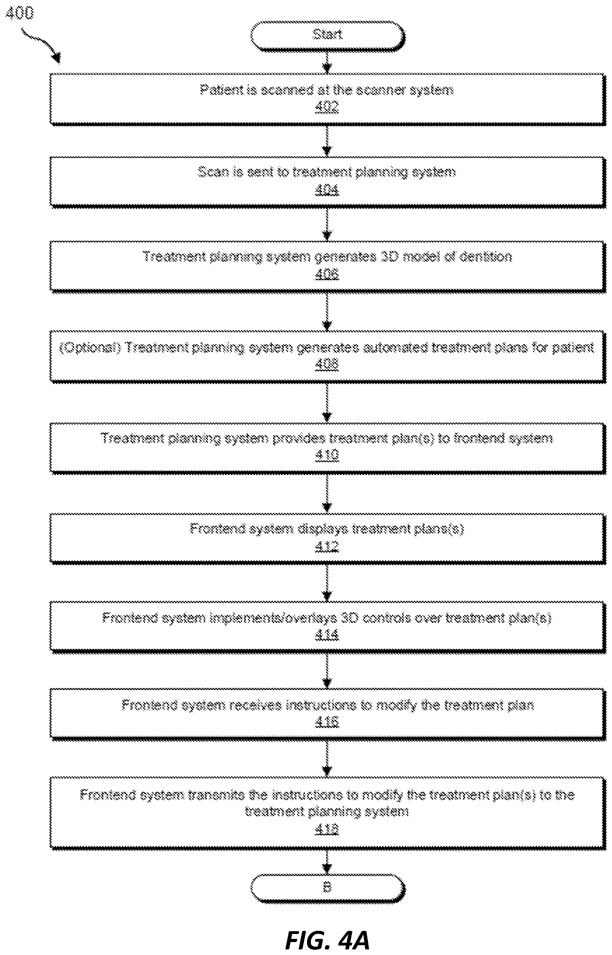

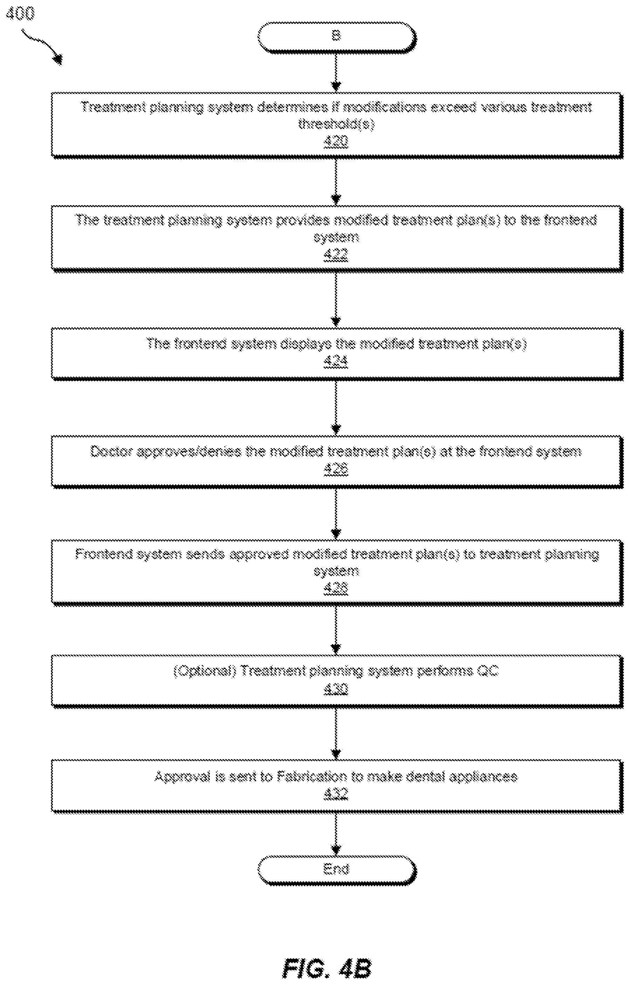

FIGS. 4A-B illustrates an example workflow 400 for automated management of clinical modifications to treatment plans according to aspects of the present disclosure. At 402, the patient is scanned at the scanner system, such as dental scanning system 220 and/or image capture system 250.

At 404, the scan is sent to a treatment planning system, which may be a backend system such as dental treatment planning system 230 and/or dental treatment simulation system 240.

At 406, the treatment planning system generates a 3D model of the scanned dentition.

Optionally at 408, the treatment planning system generates automated treatment plans for the patient.

At 410, the treatment planning system provides treatment plan(s) to a frontend system.

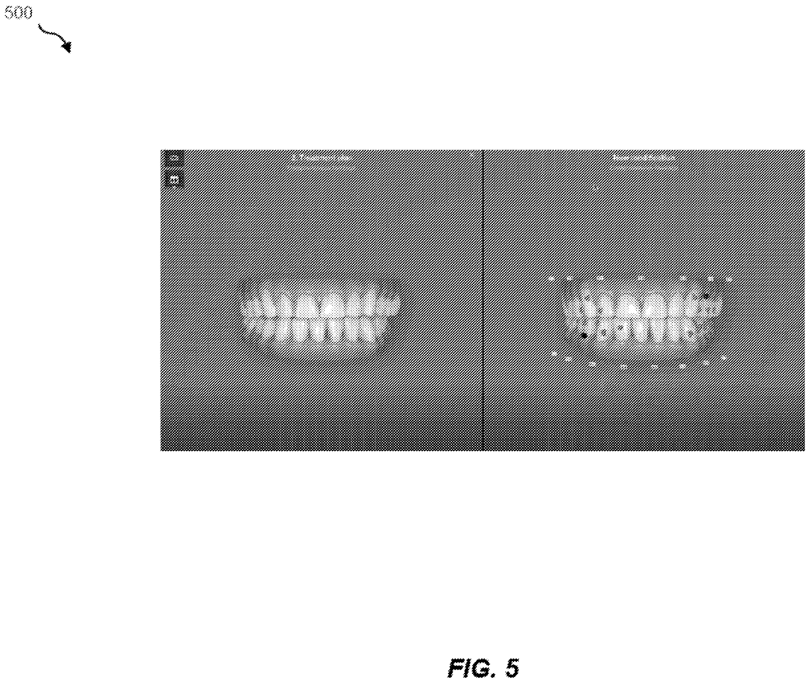

At 412, the frontend system displays the treatment plan(s). For example, FIG. 5 illustrates an example screen 500 of a frontend system displaying a treatment plan with an interface for adding modifications to the treatment plan.

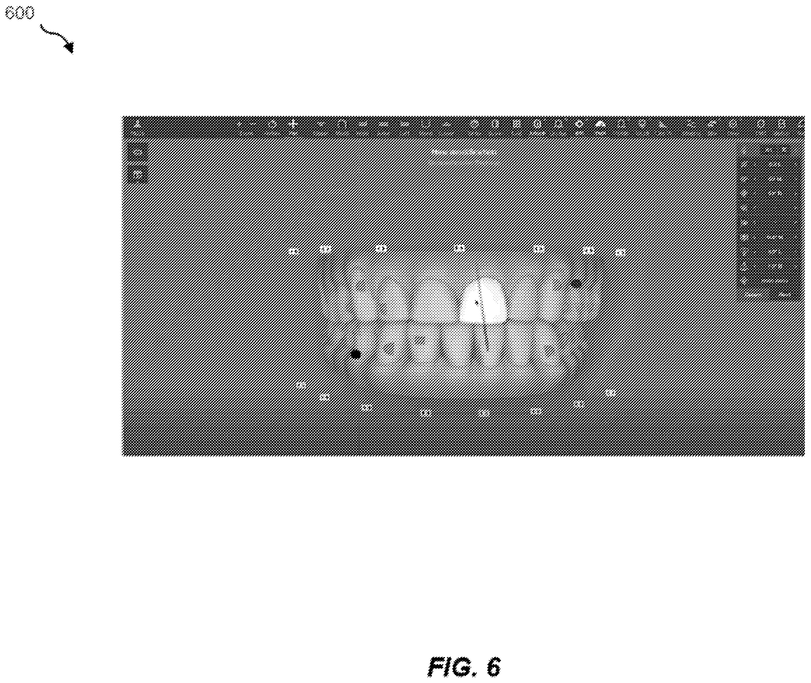

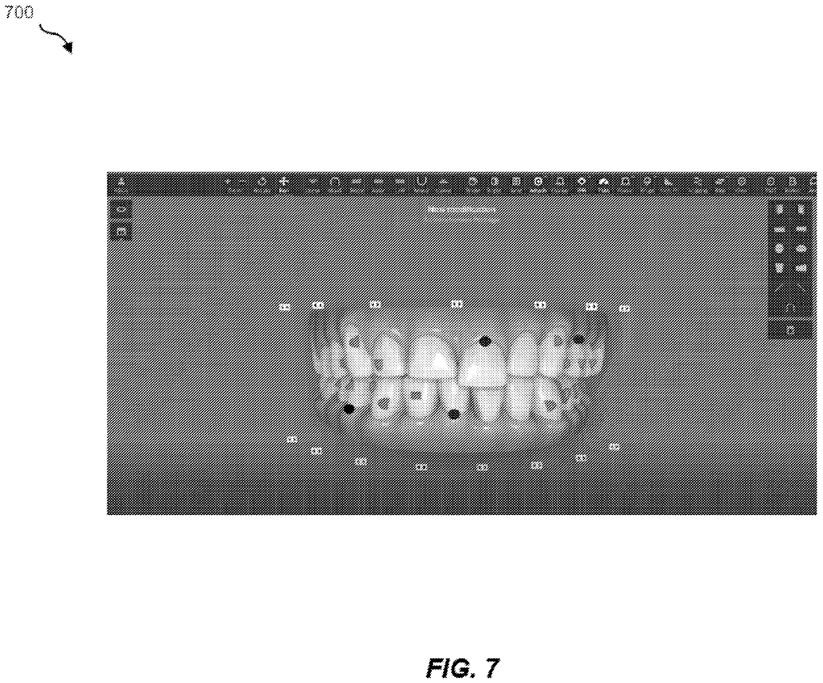

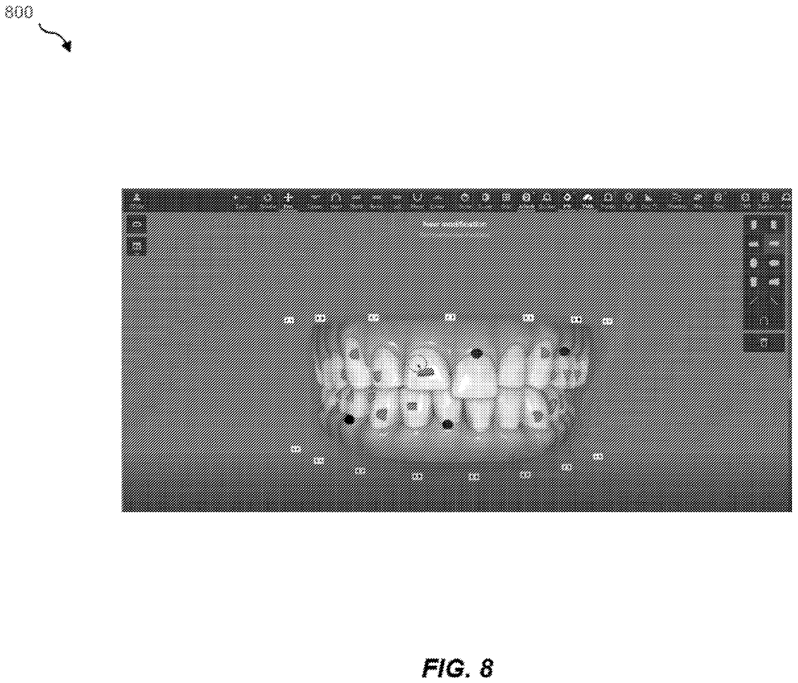

At 414, the frontend system implements and/or overlays 3D controls over the treatment plan(s). FIG. 6 illustrates an example screen 600 of the frontend system displaying 3D controls for selecting and moving a tooth. FIG. 7 illustrates an example screen 700 of the frontend system displaying 3D controls for selecting and moving an attachment. FIG. 8 illustrates an example screen 800 of the frontend system displaying 3D controls for moving the attachment, e.g., by dragging.

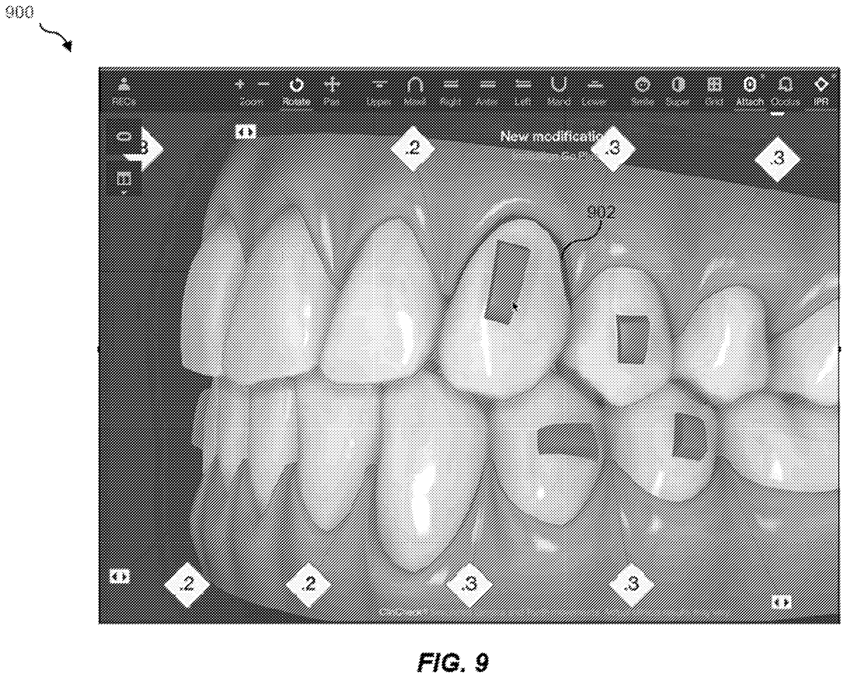

At 416, the frontend system receives instructions to modify the treatment plan through: (a) 3D controls, (b) text instructions, and/or (c) other IPL-compatible input, such as domain specific or other treatment protocols. In some examples, the frontend system may provide a warning notification or other visual indication that a proposed modification may violate or otherwise exceed certain constraints. For example, FIG. 9 illustrates an example screen 900 of the frontend system displaying a warning indicator 902. Warning indicator 902 may be, for example, overlaid onto the 3D model to designate locations, such as near the gingival line, which the attachment may not be located. Warning indicator 902 may correspond to constraint thresholds, such as a proximity threshold to the gingival line. In some examples, the proposed modification may be rejected if it exceeds warning indicator 902.

At 418, the frontend system transmits the instructions to modify the treatment plan to the treatment planning system.

At 420, the treatment planning system determines if modifications exceed various treatment thresholds. If the modifications exceed the thresholds, the treatment planning system returns notifications to the frontend system that the modifications are not acceptable. For example, the frontend system may display warning or error messages describing the exceeded thresholds. If thresholds are not exceeded, the treatment planning system translates the modifications to treatment planning steps on the 3D model and/or the treatment plan.

At 422, the treatment planning system provides the modified treatment plan(s) to the frontend system.

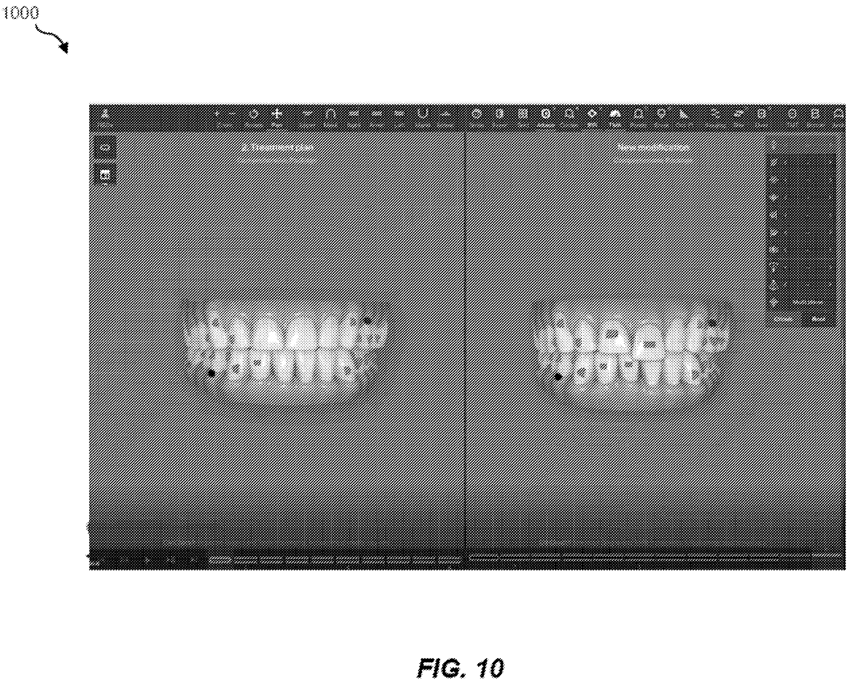

At 424, the frontend system displays the modified treatment plan. In some examples, the frontend system may display both the initial or original (e.g., unmodified) treatment plan next to the modified treatment plan. FIG. 10 illustrates an example screen 1000 of the frontend system displaying the original treatment plan and the modified treatment plan.

At 426, the doctor approves or denies the modified treatment plan at the frontend system, for example through 3D controls or text instructions.

At 428, the frontend system sends the approved modified treatment plan to the treatment planning system.

Optionally at 430, the treatment planning system performs quality control ("QC"). The QC may be performed manually or may be any other form of QC as needed.

At 432, the approval is sent to a fabrication system to make aligners, palatal expanders, and/or other dental appliances according to the approved treatment plan.

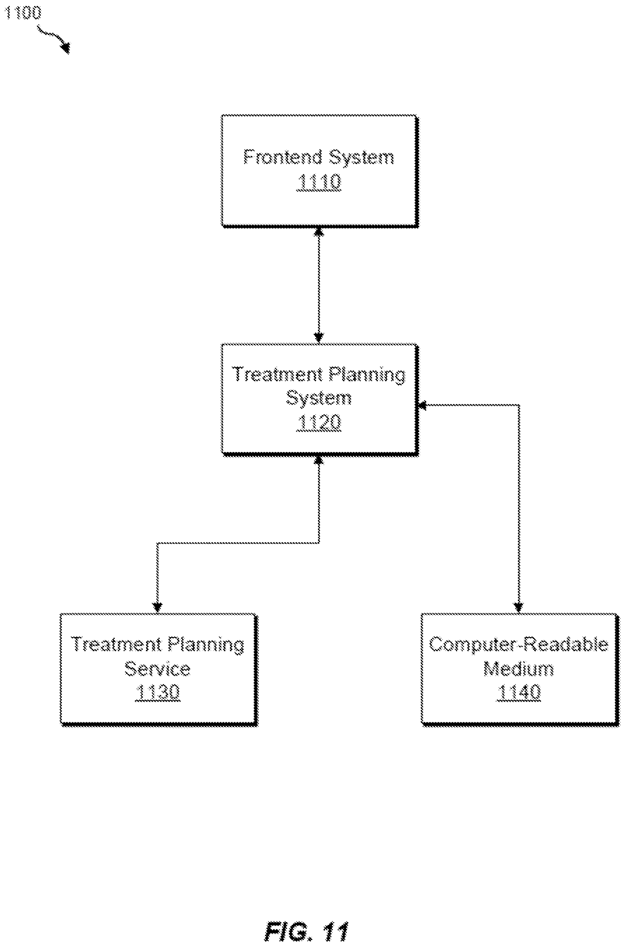

FIG. 11 illustrates a system 1100 configured to perform the processes and/or steps described herein. System 1100 may include a frontend system 1110, a treatment planning system 1120, a treatment planning service 1130, and a computer-readable medium 1140. Frontend system 1110 may include, for example, a computing device having an interface that a doctor may use to view and modify treatment plans, as described herein. Treatment planning system 1120 and treatment planning service 1130 may correspond to a backend system such as dental treatment planning system 230 and/or dental treatment simulation system 240. Treatment planning system 1120 may correspond to a service for calculating treatment plans. Treatment planning service 1130 may correspond to a service, separate from treatment planning system 1120, for managing calculation depths and files. Computer-readable medium 1140 may correspond to any computer-readable medium described herein, and may correspond to a database or datastore.

Frontend system 1110 may initialize recalculation by sending a calculate action to treatment planning system 1120, which may internally route the request to a random service pod. The service pod may create a revision in a calculating state and add it to a database. The service pod may create a concurrent delegate job and return the response with the new revision. Treatment planning system 1120 may return the response to frontend system 1110.

The delegate job in treatment planning system 1120 may start calculation by getting the revision from the database, along with additional data (e.g., ADF) from computer-readable medium 1140. Treatment planning system 1120 may put the retrieved ADF in a bucket and send the request to treatment planning service 1130. Treatment planning service 1130 may get the request and the ADF from the bucket, and recalculate the treatment plan.

Meanwhile, frontend system 1110 may poll treatment planning system 1120 iteratively until the state of the revision is completed or failed, or the calculation times out. Frontend system 1110 may send a get action to treatment planning system 1120, which may route the request to a random service pod. The service pod may read the revision from the database, and return the revision such that treatment planning system 1120 may return the response to frontend system 1110.

When the calculation is finished, treatment planning system 1120 may notify treatment planning service 1130. Treatment planning service 1130 may put the result ADF into the ADF bucket and may put the message to the topic. Treatment planning system 1120 may read the notification from the topic and send the notification to a random service pod. The service pod may check the notification. If the recalculation failed, the revision may be marked as failed. If the recalculation succeeded, the service pod may create a concurrent completion job. Treatment planning system 1120 may get the resulting ADF from the bucket and put the resulting ADF to computer-readable medium 1140. Treatment planning system 1120 may change the state of the revision to complete such that frontend system 1110 receives the revision with the next poll response.

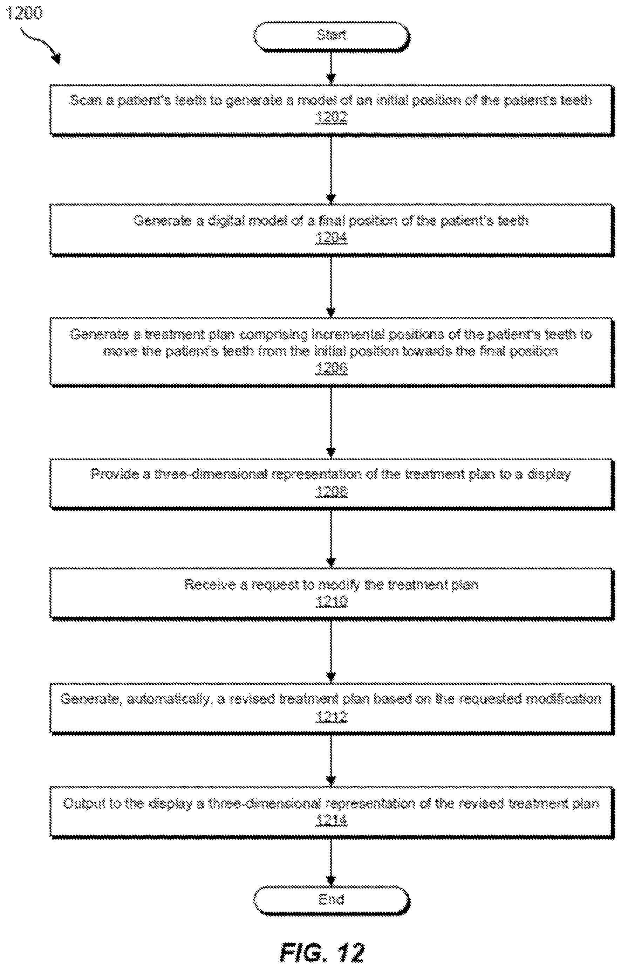

FIG. 12 shows a method 1200 for automated management of clinical modifications to treatment plans according to aspects of the present disclosure. The method 1200 may be performed by any of the systems described herein. The method 1200 may begin at 1202, by scanning a patient's teeth to generate a model of an initial position of the patient's teeth. At 1204, the systems described herein may generate a digital model of a final position of the patient's teeth. At 1206, the systems described herein may generate a treatment plan comprising incremental positions of the patient's teeth to move the patient's teeth from the initial position towards the final position.

At 1208, the systems described herein may provide a three-dimensional representation of the treatment plan to a display. In some examples, three-dimensional controls may be overlaid over the treatment plan on the display, as illustrated in FIGS. 5, 6, 7, 8, and 9. Three-dimensional controls may include controls that allow a treating professional to manipulate the three-dimensional model of the patient's dentition and treatment features. Three-dimensional controls may include controls that allow the translation and rotation of the patient's teeth, interproximal reduction modifications, translation and rotation of treatment features, including, but not limited to bite ramps and attachments, and other dentition and aligner modifications.

At 1210, the systems herein may receive a request to modify the treatment plan. In some examples, the modification to the treatment plan may be received via the three-dimensional controls. In some examples, the request to modify the treatment plan may be based on the modification received via the three-dimensional controls. In some examples, the request to modify the treatment plan may be received via text instructions or an IPL compatible input.

In some examples, the modification may be a modification to a tooth location, interproximal reduction amount, the final position of the patient's teeth, intermediate positions of the patient's teeth in one or more stages of the treatment plan, to locations of or stages used for attachments, locations of or stages used for power ridges, locations of or stages used for hooks, locations of or stages used for precision cuts, and/or locations of or stages used for interproximal reduction.

At 1212, the systems described herein may generate, automatically, a revised treatment plan based on the requested modification. In some examples, the revised treatment plan based on the requested modification may be automatically generated without manual intervention.

In some examples, generating the revised treatment plan may include determining whether or not the requested modification is clinically acceptable. The determining may be based on one or more thresholds, such as tooth movement thresholds, and/or location thresholds for aligner features. The aligner features may be one of an attachment receiving cavity location, a cut, a hook, and/or power ridges. The location threshold may be a proximity threshold with respect to a gingival line.

When the requested modification results in a potentially undesirable treatment, then the systems described herein may return an indication to the display indicating why the treatment may be potentially undesirable. Potentially undesirable treatment may include limitations in manufacturing, impacts on treatment duration (such as increases in treatment duration), requests that contradict best orthodontic treatment practices, or requests that are unlikely to work. For example, if a requested modification is not clinically acceptable, the systems described herein may return an indication to the display that the modification is not clinically acceptable. In some examples, the indication may include a visual indication of the location threshold on a display. The indication may be provided in real time or near real time, such as within about 5 minutes or about 10 minutes or less.

At 1214, the systems described herein may output to a display a three-dimensional representation of the revised treatment plan. In some examples, when the requested modification is clinically acceptable, the systems described herein may output to the display a three-dimensional representation of the revised treatment plan.

In some examples, the systems described herein may receive an approval of the modified treatment plan. The systems described herein may output instructions for fabricating a plurality of orthodontic appliances based on the modified treatment plan.

Although method 1200 is presented as a sequence of steps, in some examples, the steps of method 1200 may be repeated as needed to further modify the treatment plan. Thus, certain steps may be repeated, performed in parallel, and/or performed in a different order.

FIG. 13 is a block diagram of an example computing system 1310 capable of implementing one or more of the embodiments described and/or illustrated herein. For example, all or a portion of computing system 1310 may perform and/or be a means for performing, either alone or in combination with other elements, one or more of the steps described herein (such as one or more of the steps illustrated in FIGS. 3, 4, 11, and 12). All or a portion of computing system 1310 may also perform and/or be a means for performing any other steps, methods, or processes described and/or illustrated herein.

Computing system 1310 broadly represents any single or multi-processor computing device or system capable of executing computer-readable instructions. Examples of computing system 1310 include, without limitation, workstations, laptops, client-side terminals, servers, distributed computing systems, handheld devices, or any other computing system or device. In its most basic configuration, computing system 1310 may include at least one processor 1314 and a system memory 1316.

Processor 1314 generally represents any type or form of physical processing unit (e.g., a hardware-implemented central processing unit) capable of processing data or interpreting and executing instructions. In certain embodiments, processor 1314 may receive instructions from a software application or module. These instructions may cause processor 1314 to perform the functions of one or more of the example embodiments described and/or illustrated herein.

System memory 1316 generally represents any type or form of volatile or non-volatile storage device or medium capable of storing data and/or other computer-readable instructions. Examples of system memory 1316 include, without limitation, Random Access Memory (RAM), Read Only Memory (ROM), flash memory, or any other suitable memory device. Although not required, in certain embodiments computing system 1310 may include both a volatile memory unit (such as, for example, system memory 1316) and a non-volatile storage device (such as, for example, primary storage device 1332, as described in detail below).

In some examples, system memory 1316 may store and/or load an operating system 1340 for execution by processor 1314. In one example, operating system 1340 may include and/or represent software that manages computer hardware and software resources and/or provides common services to computer programs and/or applications on computing system 1310. Examples of operating system 1340 include, without limitation, LINUX, JUNOS, MICROSOFT WINDOWS, WINDOWS MOBILE, MAC OS, APPLE'S IOS, UNIX, GOOGLE CHROME OS, GOOGLE'S ANDROID, SOLARIS, variations of one or more of the same, and/or any other suitable operating system.

In certain embodiments, example computing system 1310 may also include one or more components or elements in addition to processor 1314 and system memory 1316. For example, as illustrated in FIG. 13, computing system 1310 may include a memory controller 1318, an Input/Output (I/O) controller 1320, and a communication interface 1322, each of which may be interconnected via a communication infrastructure 1312. Communication infrastructure 1312 generally represents any type or form of infrastructure capable of facilitating communication between one or more components of a computing device. Examples of communication infrastructure 1312 include, without limitation, a communication bus (such as an Industry Standard Architecture (ISA), Peripheral Component Interconnect (PCI), PCI Express (PCIe), or similar bus) and a network.

Memory controller 1318 generally represents any type or form of device capable of handling memory or data or controlling communication between one or more components of computing system 1310. For example, in certain embodiments memory controller 1318 may control communication between processor 1314, system memory 1316, and I/O controller 1320 via communication infrastructure 1312.

I/O controller 1320 generally represents any type or form of module capable of coordinating and/or controlling the input and output functions of a computing device. For example, in certain embodiments I/O controller 1320 may control or facilitate transfer of data between one or more elements of computing system 1310, such as processor 1314, system memory 1316, communication interface 1322, display adapter 1326, input interface 1330, and storage interface 1334.

As illustrated in FIG. 13, computing system 1310 may also include at least one display device 1324 coupled to I/O controller 1320 via a display adapter 1326. Display device 1324 generally represents any type or form of device capable of visually displaying information forwarded by display adapter 1326. Similarly, display adapter 1326 generally represents any type or form of device configured to forward graphics, text, and other data from communication infrastructure 1312 (or from a frame buffer, as known in the art) for display on display device 1324.

As illustrated in FIG. 13, example computing system 1310 may also include at least one input device 1328 coupled to I/O controller 1320 via an input interface 1330. Input device 1328 generally represents any type or form of input device capable of providing input, either computer or human generated, to example computing system 1310. Examples of input device 1328 include, without limitation, a keyboard, a pointing device, a speech recognition device, variations or combinations of one or more of the same, and/or any other input device.

Additionally or alternatively, example computing system 1310 may include additional I/O devices. For example, example computing system 1310 may include I/O device 1336. In this example, I/O device 1336 may include and/or represent a user interface that facilitates human interaction with computing system 1310. Examples of I/O device 1336 include, without limitation, a computer mouse, a keyboard, a monitor, a printer, a modem, a camera, a scanner, a microphone, a touchscreen device, variations or combinations of one or more of the same, and/or any other I/O device.

Communication interface 1322 broadly represents any type or form of communication device or adapter capable of facilitating communication between example computing system 1310 and one or more additional devices. For example, in certain embodiments communication interface 1322 may facilitate communication between computing system 1310 and a private or public network including additional computing systems. Examples of communication interface 1322 include, without limitation, a wired network interface (such as a network interface card), a wireless network interface (such as a wireless network interface card), a modem, and any other suitable interface. In at least one embodiment, communication interface 1322 may provide a direct connection to a remote server via a direct link to a network, such as the Internet. Communication interface 1322 may also indirectly provide such a connection through, for example, a local area network (such as an Ethernet network), a personal area network, a telephone or cable network, a cellular telephone connection, a satellite data connection, or any other suitable connection.

In certain embodiments, communication interface 1322 may also represent a host adapter configured to facilitate communication between computing system 1310 and one or more additional network or storage devices via an external bus or communications channel. Examples of host adapters include, without limitation, Small Computer System Interface (SCSI) host adapters, Universal Serial Bus (USB) host adapters, Institute of Electrical and Electronics Engineers (IEEE) 1394 host adapters, Advanced Technology Attachment (ATA), Parallel ATA (PATA), Serial ATA (SATA), and External SATA (eSATA) host adapters, Fibre Channel interface adapters, Ethernet adapters, or the like. Communication interface 1322 may also allow computing system 1310 to engage in distributed or remote computing. For example, communication interface 1322 may receive instructions from a remote device or send instructions to a remote device for execution.

In some examples, system memory 1316 may store and/or load a network communication program 1338 for execution by processor 1314. In one example, network communication program 1338 may include and/or represent software that enables computing system 1310 to establish a network connection 1342 with another computing system (not illustrated in FIG. 13) and/or communicate with the other computing system by way of communication interface 1322. In this example, network communication program 1338 may direct the flow of outgoing traffic that is sent to the other computing system via network connection 1342. Additionally or alternatively, network communication program 1338 may direct the processing of incoming traffic that is received from the other computing system via network connection 1342 in connection with processor 1314.

Although not illustrated in this way in FIG. 13, network communication program 1338 may alternatively be stored and/or loaded in communication interface 1322. For example, network communication program 1338 may include and/or represent at least a portion of software and/or firmware that is executed by a processor and/or Application Specific Integrated Circuit (ASIC) incorporated in communication interface 1322.

As illustrated in FIG. 13, example computing system 1310 may also include a primary storage device 1332 and a backup storage device 1333 coupled to communication infrastructure 1312 via a storage interface 1334. Storage devices 1332 and 1333 generally represent any type or form of storage device or medium capable of storing data and/or other computer-readable instructions. For example, storage devices 1332 and 1333 may be a magnetic disk drive (e.g., a so-called hard drive), a solid state drive, a floppy disk drive, a magnetic tape drive, an optical disk drive, a flash drive, or the like. Storage interface 1334 generally represents any type or form of interface or device for transferring data between storage devices 1332 and 1333 and other components of computing system 1310.

In certain embodiments, storage devices 1332 and 1333 may be configured to read from and/or write to a removable storage unit configured to store computer software, data, or other computer-readable information. Examples of suitable removable storage units include, without limitation, a floppy disk, a magnetic tape, an optical disk, a flash memory device, or the like. Storage devices 1332 and 1333 may also include other similar structures or devices for allowing computer software, data, or other computer-readable instructions to be loaded into computing system 1310. For example, storage devices 1332 and 1333 may be configured to read and write software, data, or other computer-readable information. Storage devices 1332 and 1333 may also be a part of computing system 1310 or may be a separate device accessed through other interface systems.

Many other devices or subsystems may be connected to computing system 1310. Conversely, all of the components and devices illustrated in FIG. 13 need not be present to practice the embodiments described and/or illustrated herein. The devices and subsystems referenced above may also be interconnected in different ways from that shown in FIG. 13. Computing system 1310 may also employ any number of software, firmware, and/or hardware configurations. For example, one or more of the example embodiments disclosed herein may be encoded as a computer program (also referred to as computer software, software applications, computer-readable instructions, or computer control logic) on a computer-readable medium. The term "computer-readable medium," as used herein, generally refers to any form of device, carrier, or medium capable of storing or carrying computer-readable instructions. Examples of computer-readable media include, without limitation, transmission-type media, such as carrier waves, and non-transitory-type media, such as magnetic-storage media (e.g., hard disk drives, tape drives, and floppy disks), optical-storage media (e.g., Compact Disks (CDs), Digital Video Disks (DVDs), and BLU-RAY disks), electronic-storage media (e.g., solid-state drives and flash media), and other distribution systems.

The computer-readable medium containing the computer program may be loaded into computing system 1310. All or a portion of the computer program stored on the computer-readable medium may then be stored in system memory 1316 and/or various portions of storage devices 1332 and 1333. When executed by processor 1314, a computer program loaded into computing system 1310 may cause processor 1314 to perform and/or be a means for performing the functions of one or more of the example embodiments described and/or illustrated herein. Additionally or alternatively, one or more of the example embodiments described and/or illustrated herein may be implemented in firmware and/or hardware. For example, computing system 1310 may be configured as an Application Specific Integrated Circuit (ASIC) adapted to implement one or more of the example embodiments disclosed herein.

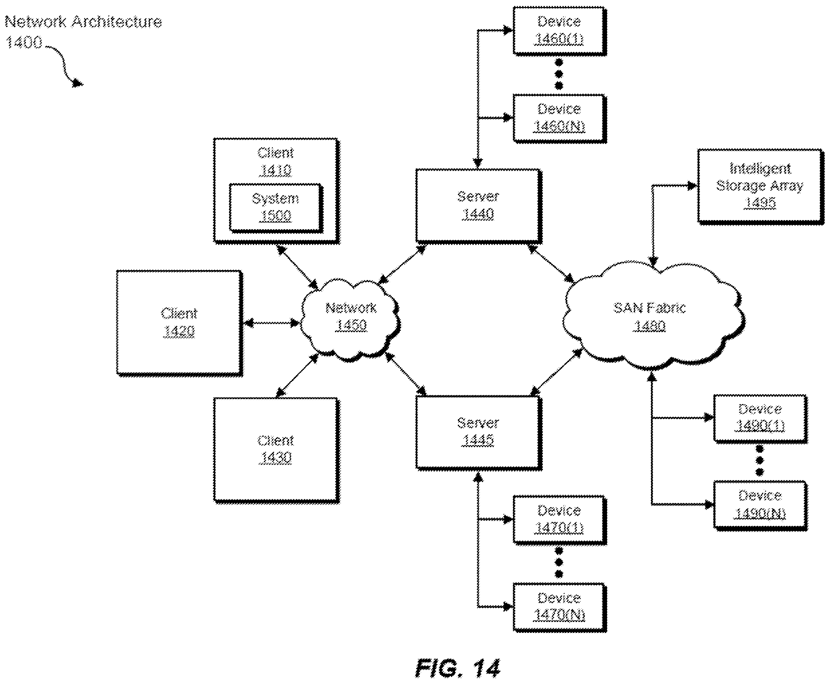

FIG. 14 is a block diagram of an example network architecture 1400 in which client systems 1410, 1420, and 1430 and servers 1440 and 1445 may be coupled to a network 1450. As detailed above, all or a portion of network architecture 1400 may perform and/or be a means for performing, either alone or in combination with other elements, one or more of the steps disclosed herein (such as one or more of the steps illustrated in FIGS. 3, 4, 11, and 12). All or a portion of network architecture 1400 may also be used to perform and/or be a means for performing other steps and features set forth in the instant disclosure.

Client systems 1410, 1420, and 1430 generally represent any type or form of computing device or system, such as example computing system 1310 in FIG. 13. Similarly, servers 1440 and 1445 generally represent computing devices or systems, such as application servers or database servers, configured to provide various database services and/or run certain software applications. Network 1450 generally represents any telecommunication or computer network including, for example, an intranet, a WAN, a LAN, a PAN, or the Internet. In one example, client systems 1410, 1420, and/or 1430 and/or servers 1440 and/or 1445 may include all or a portion of system 200 from FIG. 2, and/or system 1100 from FIG. 11.

As illustrated in FIG. 14, one or more storage devices 1460(1)-(N) may be directly attached to server 1440. Similarly, one or more storage devices 1470(1)-(N) may be directly attached to server 1445. Storage devices 1460(1)-(N) and storage devices 1470(1)-(N) generally represent any type or form of storage device or medium capable of storing data and/or other computer-readable instructions. In certain embodiments, storage devices 1460(1)-(N) and storage devices 1470(1)-(N) may represent Network-Attached Storage (NAS) devices configured to communicate with servers 1440 and 1445 using various protocols, such as Network File System (NFS), Server Message Block (SMB), or Common Internet File System (CIFS).

Servers 1440 and 1445 may also be connected to a Storage Area Network (SAN) fabric 1480. SAN fabric 1480 generally represents any type or form of computer network or architecture capable of facilitating communication between a plurality of storage devices. SAN fabric 1480 may facilitate communication between servers 1440 and 1445 and a plurality of storage devices 1490(1)-(N) and/or an intelligent storage array 1495. SAN fabric 1480 may also facilitate, via network 1450 and servers 1440 and 1445, communication between client systems 1410, 1420, and 1430 and storage devices 1490(1)-(N) and/or intelligent storage array 1495 in such a manner that devices 1490(1)-(N) and array 1495 appear as locally attached devices to client systems 1410, 1420, and 1430. As with storage devices 1460(1)-(N) and storage devices 1470(1)-(N), storage devices 1490(1)-(N) and intelligent storage array 1495 generally represent any type or form of storage device or medium capable of storing data and/or other computer-readable instructions.

In certain embodiments, and with reference to example computing system 1310 of FIG. 13, a communication interface, such as communication interface 1322 in FIG. 13, may be used to provide connectivity between each client system 1410, 1420, and 1430 and network 1450. Client systems 1410, 1420, and 1430 may be able to access information on server 1440 or 1445 using, for example, a web browser or other client software. Such software may allow client systems 1410, 1420, and 1430 to access data hosted by server 1440, server 1445, storage devices 1460(1)-(N), storage devices 1470(1)-(N), storage devices 1490(1)-(N), or intelligent storage array 1495. Although FIG. 14 depicts the use of a network (such as the Internet) for exchanging data, the embodiments described and/or illustrated herein are not limited to the Internet or any particular network-based environment.

In at least one embodiment, all or a portion of one or more of the example embodiments disclosed herein may be encoded as a computer program and loaded onto and executed by server 1440, server 1445, storage devices 1460(1)-(N), storage devices 1470(1)-(N), storage devices 1490(1)-(N), intelligent storage array 1495, or any combination thereof. All or a portion of one or more of the example embodiments disclosed herein may also be encoded as a computer program, stored in server 1440, run by server 1445, and distributed to client systems 1410, 1420, and 1430 over network 1450.

As detailed above, computing system 1310 and/or one or more components of network architecture 1400 may perform and/or be a means for performing, either alone or in combination with other elements, one or more steps of an example method for virtual care.

While the foregoing disclosure sets forth various embodiments using specific block diagrams, flowcharts, and examples, each block diagram component, flowchart step, operation, and/or component described and/or illustrated herein may be implemented, individually and/or collectively, using a wide range of hardware, software, or firmware (or any combination thereof) configurations. In addition, any disclosure of components contained within other components should be considered example in nature since many other architectures can be implemented to achieve the same functionality.

In some examples, all or a portion of system 200 from FIG. 2, and/or system 1100 from FIG. 11 may represent portions of a cloud-computing or network-based environment. Cloud-computing environments may provide various services and applications via the Internet. These cloud-based services (e.g., software as a service, platform as a service, infrastructure as a service, etc.) may be accessible through a web browser or other remote interface. Various functions described herein may be provided through a remote desktop environment or any other cloud-based computing environment.

In various embodiments, all or a portion of system 200 from FIG. 2, and/or system 1100 from FIG. 11 may facilitate multi-tenancy within a cloud-based computing environment. In other words, the software modules described herein may configure a computing system (e.g., a server) to facilitate multi-tenancy for one or more of the functions described herein. For example, one or more of the software modules described herein may program a server to enable two or more clients (e.g., customers) to share an application that is running on the server. A server programmed in this manner may share an application, operating system, processing system, and/or storage system among multiple customers (i.e., tenants). One or more of the modules described herein may also partition data and/or configuration information of a multi-tenant application for each customer such that one customer cannot access data and/or configuration information of another customer.

According to various embodiments, all or a portion of system 200 from FIG. 2, and/or system 1100 from FIG. 11 may be implemented within a virtual environment. For example, the modules and/or data described herein may reside and/or execute within a virtual machine. As used herein, the term "virtual machine" generally refers to any operating system environment that is abstracted from computing hardware by a virtual machine manager (e.g., a hypervisor). Additionally or alternatively, the modules and/or data described herein may reside and/or execute within a virtualization layer. As used herein, the term "virtualization layer" generally refers to any data layer and/or application layer that overlays and/or is abstracted from an operating system environment. A virtualization layer may be managed by a software virtualization solution (e.g., a file system filter) that presents the virtualization layer as though it were part of an underlying base operating system. For example, a software virtualization solution may redirect calls that are initially directed to locations within a base file system and/or registry to locations within a virtualization layer.

In some examples, all or a portion of system 200 from FIG. 2, and/or system 1100 from FIG. 11 may represent portions of a mobile computing environment. Mobile computing environments may be implemented by a wide range of mobile computing devices, including mobile phones, tablet computers, e-book readers, personal digital assistants, wearable computing devices (e.g., computing devices with a head-mounted display, smartwatches, etc.), and the like. In some examples, mobile computing environments may have one or more distinct features, including, for example, reliance on battery power, presenting only one foreground application at any given time, remote management features, touchscreen features, location and movement data (e.g., provided by Global Positioning Systems, gyroscopes, accelerometers, etc.), restricted platforms that restrict modifications to system-level configurations and/or that limit the ability of third-party software to inspect the behavior of other applications, controls to restrict the installation of applications (e.g., to only originate from approved application stores), etc. Various functions described herein may be provided for a mobile computing environment and/or may interact with a mobile computing environment.

In addition, all or a portion of system 200 from FIG. 2, and/or system 1100 from FIG.
11 may represent portions of, interact with, consume data produced by, and/or produce data consumed by one or more systems for information management. As used herein, the term "information management" may refer to the protection, organization, and/or storage of data. Examples of systems for information management may include, without limitation, storage systems, backup systems, archival systems, replication systems, high availability systems, data search systems, virtualization systems, and the like.

In some embodiments, all or a portion of system 200 from FIG. 2, and/or system 1100 from FIG. 11 may represent portions of, produce data protected by, and/or communicate with one or more systems for information security. As used herein, the term "information security" may refer to the control of access to protected data. Examples of systems for information security may include, without limitation, systems providing managed security services, data loss prevention systems, identity authentication systems, access control systems, encryption systems, policy compliance systems, intrusion detection and prevention systems, electronic discovery systems, and the like.

It should be appreciated that all combinations of the foregoing concepts and additional concepts discussed in greater detail below (provided such concepts are not mutually inconsistent) are contemplated as being part of the inventive subject matter disclosed herein and may be used to achieve the benefits described herein.

The process parameters and sequence of steps described and/or illustrated herein are given by way of example only and can be varied as desired. For example, while the steps illustrated and/or described herein may be shown or discussed in a particular order, these steps do not necessarily need to be performed in the order illustrated or discussed. The various example methods described and/or illustrated herein may also omit one or more of the steps described or illustrated herein or include additional steps in addition to those disclosed.

Any of the methods (including user interfaces) described herein may be implemented as software, hardware or firmware, and may be described as a non-transitory computer-readable storage medium storing a set of instructions capable of being executed by a processor (e.g., computer, tablet, smartphone, etc.), that when executed by the processor causes the processor to control perform any of the steps, including but not limited to: displaying, communicating with the user, analyzing, modifying parameters (including timing, frequency, intensity, etc.), determining, alerting, or the like. For example, any of the methods described herein may be performed, at least in part, by an apparatus including one or more processors having a memory storing a non-transitory computer-readable storage medium storing a set of instructions for the processes(s) of the method.

While various embodiments have been described and/or illustrated herein in the context of fully functional computing systems, one or more of these example embodiments may be distributed as a program product in a variety of forms, regardless of the particular type of computer-readable media used to actually carry out the distribution. The embodiments disclosed herein may also be implemented using software modules that perform certain tasks. These software modules may include script, batch, or other executable files that may be stored on a computer-readable storage medium or in a computing system. In some embodiments, these software modules may configure a computing system to perform one or more of the example embodiments disclosed herein.

As described herein, the computing devices and systems described and/or illustrated herein broadly represent any type or form of computing device or system capable of executing computer-readable instructions, such as those contained within the modules described herein. In their most basic configuration, these computing device(s) may each comprise at least one memory device and at least one physical processor.

The term "memory" or "memory device," as used herein, generally represents any type or form of volatile or non-volatile storage device or medium capable of storing data and/or computer-readable instructions. In one example, a memory device may store, load, and/or maintain one or more of the modules described herein. Examples of memory devices comprise, without limitation, Random Access Memory (RAM), Read Only Memory (ROM), flash memory, Hard Disk Drives (HDDs), Solid-State Drives (SSDs), optical disk drives, caches, variations or combinations of one or more of the same, or any other suitable storage memory.

In addition, the term "processor" or "physical processor," as used herein, generally refers to any type or form of hardware-implemented processing unit capable of interpreting and/or executing computer-readable instructions. In one example, a physical processor may access and/or modify one or more modules stored in the above-described memory device. Examples of physical processors comprise, without limitation, microprocessors, microcontrollers, Central Processing Units (CPUs), Field-Programmable Gate Arrays (FPGAs) that implement softcore processors, Application-Specific Integrated Circuits (ASICs), portions of one or more of the same, variations or combinations of one or more of the same, or any other suitable physical processor.

Although illustrated as separate elements, the method steps described and/or illustrated herein may represent portions of a single application. In addition, in some embodiments one or more of these steps may represent or correspond to one or more software applications or programs that, when executed by a computing device, may cause the computing device to perform one or more tasks, such as the method step.

In addition, one or more of the devices described herein may transform data, physical devices, and/or representations of physical devices from one form to another. Additionally or alternatively, one or more of the modules recited herein may transform a processor, volatile memory, non-volatile memory, and/or any other portion of a physical computing device from one form of computing device to another form of computing device by executing on the computing device, storing data on the computing device, and/or otherwise interacting with the computing device.

The term "computer-readable medium," as used herein, generally refers to any form of device, carrier, or medium capable of storing or carrying computer-readable instructions. Examples of computer-readable media comprise, without limitation, transmission-type media, such as carrier waves, and non-transitory-type media, such as magnetic-storage media (e.g., hard disk drives, tape drives, and floppy disks), optical-storage media (e.g., Compact Disks (CDs), Digital Video Disks (DVDs), and BLU-RAY disks), electronic-storage media (e.g., solid-state drives and flash media), and other distribution systems.

A person of ordinary skill in the art will recognize that any process or method disclosed herein can be modified in many ways. The process parameters and sequence of the steps described and/or illustrated herein are given by way of example only and can be varied as desired. For example, while the steps illustrated and/or described herein may be shown or discussed in a particular order, these steps do not necessarily need to be performed in the order illustrated or discussed.

The various exemplary methods described and/or illustrated herein may also omit one or more of the steps described or illustrated herein or comprise additional steps in addition to those disclosed. Further, a step of any method as disclosed herein can be combined with any one or more steps of any other method as disclosed herein.

The processor as described herein can be configured to perform one or more steps of any method disclosed herein. Alternatively or in combination, the processor can be configured to combine one or more steps of one or more methods as disclosed herein.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

In general, any of the apparatuses and methods described herein should be understood to be inclusive, but all or a sub-set of the components and/or steps may alternatively be exclusive, and may be expressed as "consisting of" or alternatively "consisting essentially of" the various components, steps, sub-components or sub-steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made to various embodiments without departing from the scope of the invention as described by the claims. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the invention as it is set forth in the claims.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, if more than one is, in fact, disclosed. Thus, although specific embodiments have been illustrated and described herein, any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description.

### EMBODIMENTS

1. A method of orthodontically treating teeth, the method comprising:
   generating a digital model of a final position of a patient's teeth from a scan of the patient's teeth in an initial position of the patient's teeth;
   generating a treatment plan comprising incremental positions of the patient's teeth to move the patient's teeth from the initial position towards the final position;
   providing a three-dimensional representation of the treatment plan to a display;
   receiving, in real time, a user request to modify the treatment plan and determining, in real time, that the requested user modification is within a predetermined thresholds for modifications to the treatment plan,
   generating, automatically and in real time when the user requested modification is within the predetermined threshold, a revised treatment plan based on the user requested modification; and
   outputting to the display a three-dimensional representation of the revised treatment plan.
2. The method of embodiment 1, wherein determining, in real time, that the requested user modification is within the predetermined thresholds for modifications to the treatment plan comprises determining that the user requested modification is within a predetermined threshold for space required for one or more of: attachments and power ridges, or moving teeth without collisions.
3. The method of embodiment 1, wherein generating the treatment plan comprises generating the treatment plan at a remote processor and transferring the treatment plan to a local processor that is in immediate communication with the display.
4. The method of embodiment 3, wherein generating the revised treatment plan comprises generating the treatment plan at the local processor.
5. The method of embodiment 3, further comprising transferring the revised treatment plan to the remote processor for quality review.
6. The method of embodiment 1, further comprising scanning a patient's teeth to generating a model of an initial position of the patient's teeth.
7. The method of embodiment 1, further comprising overlaying three-dimensional controls over the treatment plan on the display.
8. The method of embodiment 7, further comprising receiving the user request to modify the treatment plan via the three-dimensional controls, wherein the request to modify the treatment plan is based on the modification received via the three-dimensional controls.
9. The method of embodiment 1, wherein the user request to modify the treatment plan is received via text instructions or an IPL compatible input.
10. The method of embodiment 1, further comprising returning an indication to the display if the user request to modify the treatment plan is outside of the predetermined thresholds for modifications to the treatment plan.
11. The method of embodiment 1, wherein the predetermined thresholds for modifications to the treatment plan are tooth movement thresholds.
12. The method of embodiment 1, wherein the predetermined thresholds for modifications to the treatment plan are location thresholds for aligner features.
13. The method of embodiment 12, wherein the location threshold is a proximity threshold with respect to a gingival line.
14. The method of embodiment 12, wherein the indication includes a visual indication of the location threshold on the display.
15. The method of embodiment 12, wherein the aligner features are one of an attachment, a cut, a hook, or power ridges.
16. The method of embodiment 1, wherein the modification is one or more of: a modification to a tooth location, interproximal reduction amount, tooth shape, pantie shape, gingiva shape, the final position of the patient's teeth, intermediate positions of the patient's teeth in one or more stages of the treatment plan, to locations of or stages used for attachments, locations of or stages used for power ridges, locations of or stages used for hooks, locations of or stages used for precision cuts, or locations of or stages used for interproximal reduction.
17. The method of embodiment 1, further comprising outputting instructions for fabricating a plurality of orthodontic appliances based on the modified treatment plan.
18. The method of embodiment 1, further comprising forming one or more aligners from the modified treatment plan.
19. The method of embodiment 1, wherein generating, automatically and in real time comprises generating within 10 minutes or less of receiving the user request to modify the treatment plan.
20. A method of orthodontically treating teeth, the method comprising:
   generating a digital model of a final position of a patient's teeth from a scan of the patient's teeth in an initial position of the patient's teeth;
   generating, at a remote processor, a treatment plan comprising incremental positions of the patient's teeth to move the patient's teeth from the initial position towards the final position, and transferring the treatment plan to a local processor;
   providing a three-dimensional representation of the treatment plan to a display that is in immediate communication with the local processor;
   receiving, in real time at the remote processor, a user request to modify the treatment plan and determining, in real time at the remote processor, that the requested user modification is within a predetermined thresholds for modifications to the treatment plan,
   generating, at the remote processor and in real time when the user requested modification is within the predetermined threshold, a revised treatment plan based on the user requested modification; and
   outputting to the display a three-dimensional representation of the revised treatment plan.
21. A system for orthodontically treating teeth, the system comprising:
   one or more processors and memory comprising instructions that when executed by the one more processors causes the system to:
   generate a digital model of a final position of a patient's teeth from a scan of the patient's teeth in an initial position of the patient's teeth;
   generate a treatment plan comprising incremental positions of the patient's teeth to move the patient's teeth from the initial position towards the final position;
   provide a three-dimensional representation of the treatment plan to a display;
   receive, in real time, a user request to modify the treatment plan and determine, in real time, that the requested user modification is within a predetermined thresholds for modifications to the treatment plan,
   generate, automatically and in real time when the user requested modification is within the predetermined threshold, a revised treatment plan based on the user requested modification; and
   output to the display a three-dimensional representation of the revised treatment plan.
22. The system of embodiment 21, wherein the treatment plan comprises incremental positions of the patients teeth to move the patient's teeth from the initial position towards the final position.
23. The system of embodiment 21, wherein generating the revised treatment includes generating one or more of a revised tooth shape, restorative object shape, implant shape, location, or orientation, or auxiliary component generation.
24. The system of embodiment 21, wherein determining that the requested user modification is within the predetermined thresholds for modifications to the treatment plan comprises determining that the user requested modification is within a predetermined threshold for space required for one or more of: attachments and power ridges, or moving teeth without collisions.
25. The system of embodiment 21, wherein generating the treatment plan comprises generating the treatment plan at a remote processor and transferring the treatment plan to a local processor that is in immediate communication with the display.
26. The system of embodiment 25, wherein generating the revised treatment plan comprises generating the treatment plan at the local processor.
27. The system of embodiment 26, wherein comprising transferring the revised treatment plan to the remote processor for quality review.
28. The system of embodiment 21, wherein the instructions further cause the system to scan a patient's teeth to generating a model of an initial position of the patient's teeth.
29. The system of embodiment 21, wherein the instructions further cause the system to overlay three-dimensional controls over the treatment plan on the display.
30. The system of embodiment 29, wherein the instructions further cause the system to receive the user request to modify the treatment plan via the three-dimensional controls, wherein the user request to modify the treatment plan is based on the modification received via the three-dimensional controls.
31. The system of embodiment 21, wherein the user request to modify the treatment plan is received via text instructions or an IPL compatible input.
32. The system of embodiment 21, wherein the instructions further cause the system to return an indication to the display if the user request to modify the treatment plan is outside of the predetermined thresholds for modifications to the treatment plan.
33. The system of embodiment 21, wherein the predetermined thresholds for modifications to the treatment plan are tooth movement thresholds.
34. The system of embodiment 21, wherein the predetermined thresholds for modifications to the treatment plan are location thresholds for aligner features.
35. The system of embodiment 34, wherein the location threshold is a proximity threshold with respect to a gingival line.
36. The system of embodiment 34, wherein the indication includes a visual indication of the location threshold on the display.
37. The system of embodiment 34, wherein the aligner features are one of an attachment, a cut, a hook, or power ridges.
38. The system of embodiment 21, wherein the modification is one or more of: a modification to a tooth location, interproximal reduction amount, tooth shape, pantie shape, gingiva shape, the final position of the patient's teeth, intermediate positions of the patient's teeth in one or more stages of the treatment plan, to locations of or stages used for attachments, locations of or stages used for power ridges, locations of or stages used for hooks, locations of or stages used for precision cuts, or locations of or stages used for interproximal reduction.
39. The system of embodiment 21, wherein the instructions further cause the system to output instructions for fabricating a plurality of orthodontic appliances based on the modified treatment plan.
40. A system for orthodontically treating teeth, the system comprising:
   one or more processors and memory comprising instructions that when executed by the one more processors causes the system to:
   generate a digital model of a final position of a patient's teeth from a scan of the patient's teeth in an initial position of the patient's teeth;
   generate, at a remote processor, a treatment plan comprising incremental positions of the patient's teeth to move the patient's teeth from the initial position towards the final position, and transfer the treatment plan to a local processor;
   provide a three-dimensional representation of the treatment plan to a display that is in immediate communication with the local processor;
   receive, in real time at the remote processor, a user request to modify the treatment plan and determine, in real time at the remote processor, that the requested user modification is within a predetermined thresholds for modifications to the treatment plan,
   generate, at the remote processor and in real time when the user requested modification is within the predetermined threshold, a revised treatment plan based on the user requested modification; and
   output to the display a three-dimensional representation of the revised treatment plan.

## Claims

1. A method of orthodontically treating teeth, the method comprising:
generating a digital model of a final position of a patient's teeth from a scan of the patient's
teeth in an initial position of the patient's teeth;
generating, at a remote processor, a treatment plan comprising incremental positions of the patient's teeth to move the patient's teeth from the initial position towards the final position, and transferring the treatment plan to a local processor;
providing a three-dimensional representation of the treatment plan to a display that is in immediate communication with the local processor;
receiving, in real time at the remote processor, a user request to modify the treatment plan and determining, in real time at the remote processor, that the requested user modification is within a predetermined thresholds for modifications to the treatment plan,
generating, at the remote processor and in real time when the user requested modification is within the predetermined threshold, a revised treatment plan based on the user requested modification; and
outputting to the display a three-dimensional representation of the revised treatment plan.

2. A method of orthodontically treating teeth, the method comprising:
generating a digital model of a final position of a patient's teeth from a scan of the patient's teeth in an initial position of the patient's teeth;
generating a treatment plan comprising incremental positions of the patient's teeth to move the patient's teeth from the initial position towards the final position;
providing a three-dimensional representation of the treatment plan to a display;
receiving, in real time, a user request to modify the treatment plan and determining, in real time, that the requested user modification is within a predetermined thresholds for modifications to the treatment plan,
generating, automatically and in real time when the user requested modification is within the predetermined threshold, a revised treatment plan based on the user requested modification; and
outputting to the display a three-dimensional representation of the revised treatment plan.

3. The method of claim 2, wherein determining, in real time, that the requested user modification is within the predetermined thresholds for modifications to the treatment plan comprises determining that the user requested modification is within a predetermined threshold for space required for one or more of: attachments and power ridges, or moving teeth without collisions, and/or wherein generating the treatment plan comprises generating the treatment plan at a remote processor and transferring the treatment plan to a local processor that is in immediate communication with the display, and optionally wherein generating the revised treatment plan comprises generating the treatment plan at the local processor, or further comprising transferring the revised treatment plan to the remote processor for quality review.

4. The method of claim 2, further comprising scanning a patient's teeth to generating a model of an initial position of the patient's teeth, and/or further comprising overlaying three-dimensional controls over the treatment plan on the display, optionally further comprising receiving the user request to modify the treatment plan via the three-dimensional controls, wherein the request to modify the treatment plan is based on the modification received via the three-dimensional controls.

5. The method of claim 2, wherein the user request to modify the treatment plan is received via text instructions or an IPL compatible input, and/or further comprising returning an indication to the display if the user request to modify the treatment plan is outside of the predetermined thresholds for modifications to the treatment plan, and/or wherein the predetermined thresholds for modifications to the treatment plan are tooth movement thresholds.

6. The method of claim 2, wherein the predetermined thresholds for modifications to the treatment plan are location thresholds for aligner features, optionally wherein the location threshold is a proximity threshold with respect to a gingival line, or wherein the indication includes a visual indication of the location threshold on the display, or wherein the aligner features are one of an attachment, a cut, a hook, or power ridges.

7. The method of claim 2, wherein the modification is one or more of: a modification to a tooth location, interproximal reduction amount, tooth shape, pontic shape, gingiva shape, the final position of the patient's teeth, intermediate positions of the patient's teeth in one or more stages of the treatment plan, to locations of or stages used for attachments, locations of or stages used for power ridges, locations of or stages used for hooks, locations of or stages used for precision cuts, or locations of or stages used for interproximal reduction, and/or further comprising outputting instructions for fabricating a plurality of orthodontic appliances based on the modified treatment plan, and/or further comprising forming one or more aligners from the modified treatment plan, and/or wherein generating, automatically and in real time comprises generating within 10 minutes or less of receiving the user request to modify the treatment plan.

8. A system for orthodontically treating teeth, the system comprising:
one or more processors and memory comprising instructions that when executed by the one more processors causes the system to:
generate a digital model of a final position of a patient's teeth from a scan of the patient's teeth in an initial position of the patient's teeth;
generate a treatment plan comprising incremental positions of the patient's teeth to move the patient's teeth from the initial position towards the final position;
provide a three-dimensional representation of the treatment plan to a display;
receive, in real time, a user request to modify the treatment plan and determine, in real time, that the requested user modification is within a predetermined thresholds for modifications to the treatment plan,
generate, automatically and in real time when the user requested modification is within the predetermined threshold, a revised treatment plan based on the user requested modification; and
output to the display a three-dimensional representation of the revised treatment plan.

9. The system of claim 8, wherein the treatment plan comprises incremental positions of the patients teeth to move the patient's teeth from the initial position towards the final position, and/or wherein generating the revised treatment includes generating one or more of a revised tooth shape, restorative object shape, implant shape, location, or orientation, or auxiliary component generation, and/or wherein determining that the requested user modification is within the predetermined thresholds for modifications to the treatment plan comprises determining that the user requested modification is within a predetermined threshold for space required for one or more of: attachments and power ridges, or moving teeth without collisions.

10. The system of claim 8, wherein generating the treatment plan comprises generating the treatment plan at a remote processor and transferring the treatment plan to a local processor that is in immediate communication with the display, optionally wherein generating the revised treatment plan comprises generating the treatment plan at the local processor, further optionally wherein comprising transferring the revised treatment plan to the remote processor for quality review.

11. The system of claim 8, wherein the instructions further cause the system to scan a patient's teeth to generating a model of an initial position of the patient's teeth, and/or wherein the instructions further cause the system to overlay three-dimensional controls over the treatment plan on the display, and optionally wherein the instructions further cause the system to receive the user request to modify the treatment plan via the three-dimensional controls, wherein the user request to modify the treatment plan is based on the modification received via the three-dimensional controls.

12. The system of claim 8, wherein the user request to modify the treatment plan is received via text instructions or an IPL compatible input, and/or wherein the instructions further cause the system to return an indication to the display if the user request to modify the treatment plan is outside of the predetermined thresholds for modifications to the treatment plan, and/or wherein the predetermined thresholds for modifications to the treatment plan are tooth movement thresholds.

13. The system of claim 8, wherein the predetermined thresholds for modifications to the treatment plan are location thresholds for aligner features, and optionally wherein the location threshold is a proximity threshold with respect to a gingival line, or wherein the indication includes a visual indication of the location threshold on the display, or wherein the aligner features are one of an attachment , a cut, a hook, or power ridges.

14. The system of claim 8, wherein the modification is one or more of: a modification to a tooth location, interproximal reduction amount, tooth shape, pontic shape, gingiva shape, the final position of the patient's teeth, intermediate positions of the patient's teeth in one or more stages of the treatment plan, to locations of or stages used for attachments, locations of or stages used for power ridges, locations of or stages used for hooks, locations of or stages used for precision cuts, or locations of or stages used for interproximal reduction, and/or wherein the instructions further cause the system to output instructions for fabricating a plurality of orthodontic appliances based on the modified treatment plan.

15. A system for orthodontically treating teeth, the system comprising:
one or more processors and memory comprising instructions that when executed by the one
more processors causes the system to:
generate a digital model of a final position of a patient's teeth from a scan of the patient's teeth in an initial position of the patient's teeth;
generate, at a remote processor, a treatment plan comprising incremental positions of the patient's teeth to move the patient's teeth from the initial position towards the final position, and transfer the treatment plan to a local processor;
provide a three-dimensional representation of the treatment plan to a display that is in immediate communication with the local processor;
receive, in real time at the remote processor, a user request to modify the treatment plan and determine, in real time at the remote processor, that the requested user modification is within a predetermined thresholds for modifications to the treatment plan,
generate, at the remote processor and in real time when the user requested modification is within the predetermined threshold, a revised treatment plan based on the user requested modification; and
output to the display a three-dimensional representation of the revised treatment plan.
